*Lepetit*  Patent and Trademark
Department
Via R. Lepetit, 34
21040 Gerenzano (Varese)
Italia
Telefono: (+392) 96191
Telex: 331165
Telefax: (+392) 96193468

Gruppo Lepetit spa
Sede legale:
20159 Milano
Via G. Murat, 23
Telefono: (02) 2777.1
Telex: 321486
Casella Postale 16184
20100 Milano

Anno di fondazione 1868
Capitale sociale
L. 29.363.000.000
interamente versato
C.C.I.AA.:
Milano n. 95669
Tribunale di Milano:
Reg. Soc. 22049

Codice Fiscale,
Partita IVA
00795960152

EPA EPO·OEB
DG 1
Recu:
**1 8 -11- 1988**
05 | | ANL ... UN.

EUROPEAN PATENT OFFICE
Erhardtstrasse 27
D-8000 München 2
Germania R.F.

Our ref: LPE 657 FM/Sm                     November 10, 1988
          E-107

Re: European Patent Application No. 88109800.9
    filed on June 20, 1988
    GRUPPO LEPETIT SpA

---

Dear Sirs,

We would like that the following amendments are entered
in the text on file according to R. 88:

-       Page 72, line 3: the reference to "Table 2" should
        be deleted. There is no "Table 2" in the application.

-       Page 81, line 29: the reference to "Table 3 below" is
        directed to the table on page 83. Thus, it should be
        amended to read "the table below"
        This amendment is evident in the sense of R. 88 in
        view of the context.

-       Page 78, first line: the term "PRT" is a word
        without any sense in the context and should be deleted.

Amended pages, 72, 78 and 81 are enclosed in triplicate.

We are also enclosing EPO Form 1037 with an addressed envelope
for acknowledgement of receipt of this letter.

With many thanks,

Sincerely yours,

Francesco Macchetta
Patent & Trademark Dept.
GA 1745


Encls: EPO Form 1037 with an addressed envelope
       Amended pages 72, 78 and 81

Europäisches Patentamt

European Patent Office

Office européen des brevets

(11) Publication number: **0 303 028**
**A1**

(19)

(12) **EUROPEAN PATENT APPLICATION**

(21) Application number: **88109800.8**

(22) Date of filing: **20.06.88**

(51) Int. Cl.⁴: **C12N 15/00 , C12N 9/72 ,**
**C07H 21/04 , C12N 5/00 ,**
**A61K 37/54**

A request for correction of the originally filed description has been filed pursuant to Rule 88 EPC. A decision on the request will be taken during the proceedings before the Examining Division (Guidelines for Examination in the EPO, A-V, 2.2).

(30) Priority: **10.08.87 GB 8718877**
**24.03.88 US 173337**

(43) Date of publication of application:
**15.02.89 Bulletin 89/07**

(84) Designated Contracting States:
**AT BE CH DE ES FR GB GR IT LI LU NL SE**

(71) Applicant: **GRUPPO LEPETIT S.P.A.**
**23, Via G. Murat**
**I-20159 Milano(IT)**

(72) Inventor: **Cassani, Giovanni**
**3, Via Vittadini**
**I-27100 Pavia(IT)**
Inventor: **Robbiati, Federico Maria**
**141, Via Porpora**
**I-20131 Milano(IT)**
Inventor: **Blasi, Francesco**
**298, Via Posillipo**
**I-80123 Napoli(IT)**
Inventor: **Nolli, Marialuisa**
**34, Via C. Riboldi**
**I-27100 Pavia(IT)**
Inventor: **Corti, Angelo**
**8, Via Leonardo da Vinci**
**I-24052 Azzano S. Paolo Bergamo(IT)**

(54) **Method for preparing single chain urokinase.**

(57) This invention relates to a method for producing single chain pro-urokinase (hereinbelow referred to also as pro-uPA or scu-PA). More particularly, it relates to a method for producing pro-uPA which comprises recovering the uPA gene from a human genomic library, inserting it into an expression vector, introducing the obtained plasmid vector DNA into an animal cell to obtain a transformant capable of producing the coded protein and recovering the glycosylated pro-uPA from said animal cell.

EP 0 303 028 A1

## METHOD FOR PREPARING SINGLE CHAIN UROKINASE

This invention relates to a method for producing single chain pro-urokinase (hereinbelow referred to also as pro-uPA or scu-PA). More particularly, it relates to a method for producing pro-uPA which comprises recovering the uPA gene from a human genomic library, inserting it into an expression vector, introducing the obtained plasmid vector DNA into an animal cell to obtain a transformant capable of producing the coded protein and recovering the glycosylated pro-uPA from said animal cell.

A further object of the present invention is represented by a human uPA promoter derivative that may control the transcription of a gene, like the urokinase gene, with high efficiency. Also included in the present invention are new eukaryotic expression vectors in which a cDNA or a gene is placed under the transcriptional control of the human uPA promoter or a derivative thereof and the expressing cells transformated therewith.

Another preferred embodiment of this invention is represented by an "expression cassette" which contains the human uPA promoter or a derivative thereof, the human uPA transcription initiator (i.e. the so-called TATA box), and, conveniently, a polylinker.

Urokinase (uPA) is a plasminogen activator, obtainable from human urines, which is used in the treatment of various forms of thrombosis or embolic diseases.

Urokinase production has been based on the purification of the enzyme essentially from human urine. With the development of cell culture technology and the availability of specific anti-urokinase antibodies (monoclonal as well as polyclonal antibodies), it has been shown that uPA is an activation product of cell-produced pro-uPA (Wun et al, 1982, J. Biol. Chem. 257,7262-7268; Nielsen et al., 1982, Biochemistry 21, 6410-6415). Pro-uPA is a single-chain protein (sc-uPA), while uPA is a two-chain protein (tc-uPA). Conversion of scu-PA into tcu-PA depends on the cleavage of a single peptide bond of scu-uPA (Verde et al, 1984; Proc. Natl. Acad. Sci. USA 81; 4727-4731; (1984); Günzler et al, Hoppe S. Zschrf. Physiol. Chem. 363, 1155-1165, 1982).

This specific cleavage of single chain pro-uPA results in over 50 fold increase in specific activity and is obtained with proteolytic enzymes such as trypsin or plasmin. In particular, plasmin appears to be responsible of this transformation also in vivo.

An inactive form of plasminogen activator was identified in human embryonic kidney cell cultures by C. Nolan already in 1977 et al., (Biochim Biophys Acta 1977; 496,384-400). This substance, that the authors considered to be a pro-activator form of urokinase, was activated by trypsin without change in molecular weight and, once activated, was inhibited by rabbit antibody to human urokinase. The inactive "pro-activator" was separated from the active form by means of an affinity chromatography on benzamidine-Sepharose.

A single-chain plasminogen activator having molecular weight of about 56,000, a specific activity of 40,000-50,000 CTA units/mg on a fibrin plate and with a high affinity for fibrin is described in European patent 40238.

European Patent Application Publ. No. 92182 deals with the preparation of urokinase derivatives via recombinant DNA technology. In particular it discloses the purification and characterization of low molecular weight urokinase (about 30,000 daltons) obtained by recombinant DNA techniques in a procariotic host (E.Coli).

Another European Patent Application, EP-A-37687, describes a deoxyribonucleic acid segment which allegedly codes for a plasminogen activator having the properties of human urokinase and a method for expressing this DNA sequence in certain bacteria. A method for producing glycosylated single-chain urokinase in animal cells by recombinant cDNA technology using a cDNA obtained from mRNA of an established human kidney-derived cell line is disclosed in European Patent Application publication No. 154272. Riccio et al, in Nucleic Acid Research, 13, 2759 (1985) describe the sequence of a fragment of the human uPA "promoter" region of about 800 bp containing the 5' flanking region.

As anticipated above, the present invention encompasses a method for producing pro-uPA by recombinant DNA technology in animal cells which comprises inserting a DNA vector which contains a DNA fragment of human genomic origin having the coding region but not the regulatory region of the human urokinase gene into a suitable animal cell to produce, upon culturing, a human-type pro-uPA having the same aminoacid sequence as the naturally occurring pro-uPA.

The DNA fragment of human genomic origin containing the uPA coding region is obtained by a human genomic library in a suitable vector, such as a library prepared in bacteriophage lambda or in cosmids according to known per se techniques, after screening the library with the proper DNA probe represented by a portion of the gene itself or a synthetic DNA fragment of a suitable length prepared on the basis of the known coding sequence or a cDNA derived probe.

Conveniently, this uPA coding fragment is the 7.3 kb fragment obtained by Smal digestion of the cloned DNA.

A DNA segment like this will be referred to in the description and claims as "open reading frame" (ORF), to mean a genomic DNA sequence including the transcription-translation unit of uPA mRNA, possibly interrupted by introns and preferably including 5' and 3' untranslated regions or a part thereof which confers translation efficiency or mnRNA stability. A preferred example of ORF is a sequence defined as above which is interrupted by 10 introns.

Expression vectors that can be used in the method of the invention are any of the known vectors used to transfect animal cells and in particular mammalian cells. Essentially, they include a sequence which regulates the mRNA translation, such as a promoter, and a polyadenylation site. In some cases they include also a eukaryotic origin of replication. In particular, the promoter region may be any of the known ones which may be used in animal cells. Heterologous promoters may be obtained from mammalian virus such as retroviruses (e.g. RSV), SV 40 (early or late) and adenovirus (early or late). Alternatively, regulable promoters of animal or human origin may be used. Examples of these regulable promoters are those from mouse or human metallothioneins I and II, heat shock proteins, etc which are found in reference publications such as Hamer et al, Eukaryotic Viral Vectors, Y. Gluzman ed, 1983, 7-12, Mayo et al, Cell, 29, 99-108 (1982) and Korin et al, Nature, 308, 513-519 (1984).

In addition to those regulable promoters, it has been also found that the human uPA promoter region or a derivative thereof may be suitably used as an efficient promoter for expressing a gene put under its control in mammalian cells. The human uPA promoter region is obtained from a human genomic library (see above) by Smal digestion and contains about 2.38 kb, from about -2353 bp to about + 29 bp on the human uPA gene.

This promoter and its derivatives can be induced by different agents such as phorbol esters to express the uPA gene at higher levels.

Not only this "native" human uPA promoter can be used to express a gene in a suitable mammalian cell system but any of its derivatives obtained by deletion, insertion or mutation which maintain the original regulatory capability or even possess an improved regulatory capability can suitably be used in the method of the invention. In particular, deletion derivatives of the uPA promoter region are preferred. Representative examples of deletion derivatives are those obtained by treating the uPA promoter with OxaNI or EcoRV and subsequent ligation of the obtained fragments. In particular by digesting with OxaNI a deletion derivative is obtained which misses a 0.6 kb fragment between about -1827 bp and -1202 bp, while by digesting with OxaNI and EcoRV a deletion derivative is obtained which misses a 1.29 kb fragment between about -1827 bp and -537 bp.

It has also been found that these deletion derivatives are capable of driving the expression of the regulated gene with comparable and even higher yields than usual viral promoters, such as RSV, which are commonly considered very efficient promoter systems. Even if the preferred gene to be expressed under the uPA promoter or a derivative thereof is the uPA gene, this promoter may be used to express any known gene or coding sequence in mammalian cells.

As for the polyadenylation site, it may be any of the known ones, such as one associated with the promoter region, native to the uPA gene or from another mammalian gene. Once an expression cassette has been prepared containing a promoter region, a transcription initiator, and conveniently, a polylinker, it may be joined to the chosen gene for transformation into an animal cell. In general, the specific order in which the parts are brought together is not critical, so that the flanking regions might be first bound to a replication system including a marker or other regions, such as enhancers, transcriptionl regulatory regions, or the like, prior to insertion of the gene.

The manner in which the various fragments are brought together depends on a number of factors related to ease of construction, choice of restriction sites, availability of particular fragments, and the like, which ultimately are within the choice capability of the man skilled in the art.

The choice of the replication system depends, to a certain extent, on whether a transient or stable expression is desired. For transient expression, episomal elements based on viral sequence, (such as SV40) containing an origin of replication are used. Stable expression can be achieved using other episomal elements (e.g. bovine papilloma virus based vectors) or sequences integrated into the genome. A number of these viral sequences have been joined to markers, such as the genes gpt, neo and dhfr. These markers allow for selection of cells containing the vector and for amplification of the integrated foreign sequences. To ultimately enhance the production of the desired substance, i.e. human pro-uPA, it may be convenient to co-transform the cells with a first construct containing the uPA expression cassette and a second independent construct containing the selectable marker.

3

Alternatively, the gene may be amplified by preparing a tandem construct where the uPA cassette is in tandem with an expression cassette of a gene such as dhfr.

The host cells may be any animal cell, preferably a mammalian or avian cell, which can produce pro-uPA in a recoverable amount upon transformation with a uPA expression vector. Examples of these cells, which expressely include cells of human origin, encompass the known and established cell lines which may or may not produce pro-uPA in recoverable amounts before transformation, such as LB6, a mouse fibroblastoid cell line derived from parental L cells (Corsaro C.M. and Pearson L.M. Enhancing the efficiency of DNA-mediated gene transfer in mammalian cells, Somatic cell genetics 7, 603-616, 1981); CHO, chinese hamster ovary (Kao F.T., Puck F., Proc. Natl. Acad. Sci., 60, 1275-1281, 1968) and A 431 human epidermoid carcinoma cell line (Fabricant R.N., De Larco J. E., and Todaro G.F., Nerve growth factor receptors on human melanoma cells in culture, Proc. Natl. Acad. Sci. USA 74, 565-569, 1977). The first two cell lines mentioned above (LB6 and CHO) do not produce pro-uPA while the last one (A 431) produces it. In general, established cells lines such as CHO, COS, LTK, Hela and CN-1 may be suitably used in the process of the invention.

Upon repeated recloning of a uPA producing mono-layer CHO cell line, derived cell lines which retain the uPA production capability can be obtained which are also capable of self-replication in suspension. The advantage of such cell lines in terms of easiness of cultivation and higher production yields per fermentation mass unit are evident to the man skilled in the art. These genomic uPA DNA transformed, CHO cell lines capable of growth in suspension in a cultivation medium represent a preferred embodiment of the present invention.

The expression vector may be introduced into the host by any convenient means such as microinjection, DEAE-dextran mediated transfection, calcium phosphate precipitated DNA transfection and electroporation (see Banerji, J. et al., Cell 33, 729-740 (1983); Graham F.L. and Van der Eb A.J., Virology, 52, 456-467 (1983) and Potter H. et al., Proc. Natl. Acad. Sci. USA, 81, 7161-7165 (1984)).

After transfection, the cells are grown in an appropriate medium, such as Dulbecco's modified Eagle medium (DMEM) containing 10% to 20% inactivated fetal calf serum, in which these cells are stably maintained and then the appropriate selection agent e.g. an antibiotic, is introduced.

The selected cells are repeatedly cloned and their production of pro-uPA/uPA is evaluated by the usual assays such as the fibrinolytic assay or the immunoamidolytic assay (IAA). The producing clones are then mass cultivated to produce substantial amounts of pro-uPA.

In mass cultivation, a preferred embodiment is represented by the addition of aprotinin. The result of the addition of this substance in the original medium or during cultivation is that pro-uPA is recovered in higher amounts. Typically aprotinin is added at a concentration of 10-50 IU/ml and most preferably 20-25 IU/ml.

Depending upon whether the leader sequence has been retained and the product is capable of secretion, the medium may be continuously or repetitively exchanged and the pro-uPA isolated from the medium. Where the leader is not retained and product is maintained intracellularly, the cells may be harvested, killed and lysed, and the product isolated. For isolation and purification, the usual techniques may be applied such as affinity chromatography, HPLC, reverse-phase HPLC, FPLC, electrophoresis, extraction, precipitation etc.

It has been surprisingly found that the scu-PA substances of the invention possess a different glycosylation on the B-chain, while retaining the same protein structure and composition of scu-PA from human urine as well as the same type of biological activity. In fact, while the A-chain of these substances show the same eletrophoretic mobility of urinary obtained scu-PA, their B chains show an appreciable difference in electrophoretic mobility corresponding approximately to an increased apparent molecular weight of 1000-2000. These differences can be evaluated by assaying with specific glycosidases such as glycopeptidase F which removes all the N-linked sugars from a glycoprotein or neurominidase which removes the terminal sialic acid residues from glycoproteins.

It can be noted that the scu-PA or tcu-PA protein obtained from the scuPA/tcu-PA's of the invention by removing all the N-terminal sugars with glycopeptidase F have the same electrophoretic mobility as the human urine obtained scu-PA or tcu-PA, treated in the same way, while by removing the terminal sialic acid residues from the scu-PA/tcu-PA's of the invention a glycoprotein is obtained which still has a different electrophoretic mobility (namely a reduced mobility) when compared, side-by-side, with human urine derived scu-PA/tcu-PA treated in the same way.

The biological activity of the scu-PA's obtained according to the present invention can be evaluated by the conventional in vitro or in vivo assays. An example of in vitro tests is represented by the so-called fibrin-plate method wherein the fibrinolytic activity of the scu-PA obtained according to the present invention is evaluated on a fibrin gel obtained by polymerization of fibrinogen with thrombin.

An in vitro text which can be used to correlate in vitro the features of the scu-PA's of the invention with those of urine-obtained or cell-obtained scu-PA's is a kinetic analysis on the activation of the scu-PA's to tcu-PA's by plasmin.

Another example of an in vitro test is represented by a kinetic analysis on the hydrolysis of a synthetic substate or of the activation of human plasminogen by plasmin-activated scu-PA.

A further in vitro experiment is represented by a study on the inhibition of the fibrinolytic activity of plasmin-activated scu-PA (i.e. a tcu-PA compound of the invention) on a specific synthetic substrate by means of a known human plasminogen activator inhibitor such as the endothelial one (PAI-1).

All these experiments are highly significant to show the thrombolytic activity of the tested substances and are predictive of therapeutic applicability.

The scu-PA/tcu-PA compounds of the invention show the same type of amidolytic and fibrinolytic activity of scu-PA/tcu-PA of human urine origin in these assays.

An in vivo model on experimental thrombus in rabbit that can be used to evaluate the thrombolytic activity of the compounds of the invention is described by D. Collen et al, in J. Clin. Inv. 1983, 73, 368-376.

Briefly, a segment of the external jugular vein of anesthetized rabbits is isolated with vascular clamps and its volume is measured. A thrombin solution followed by fresh blood containing tagged human fibrinogen is then introduced into this emptied vein segment. The blood clot which forms is left to age for a predetermined period of time (conveniently 30 min) before removing the clamps and determining the amount of tagged substance. Via a marginal ear vein the plasminogen activator to be tested is introduced by infusion. After a given time, the vessel segment containing the thrombus is sutured at its end and its content in tagged substance determined. The degree of thrombolysis is calculated as the percent ratio betweeen the amounts of the residual and the initial tagged substance.

The scu-PA compounds obtained according to the process of the invention possess the same biological properties of urine isolated scu-PA and therefore may be used for the same therapeutical indications, i.e. mainly as thrombolytics or in the treatment of embolic diseases. To this end, it can be used as such or preferably formulated according to known per se techniques which are described in reference books such as Remington's Pharmaceutical Sciences, 17th Edition, 1985, Mack Publishing Co. In particular, they can be formulated analogously to the current formulation of uPA or scu-PA.

A preferred therapeutic indication is therefore as thrombolytics, possibly in the early stages of infarction or embolism, such as in heart infarction, pulmonary embolism or peripheral arterial embolism. The route of administration is preferably e.v., and the mode of administration can be in bolus or by perfusion. Preferably, an e.v. bolus administration of a thrombolytically effective amount of a compound of the invention is followed, if necessary, by e.v. perfusion of a lower dosage for a relatively short period of time. The daily dosage varies greatly with the patient age, size, condition and for infarction and related therapy it can be indicatively in the range of 10-40 mg/die, preferably 15-30 mg/die and most preferably 20 mg/die for a normal (70 kg) human patient. As known in the art, in this particular therapeutic field the appropriate dosage and administration scheme is oftentimes decided by the physician on a case by case basis. Patients, according to the present invention are warm-blooded animals, preferably mammals and most preferably humans. The dosage unit, which contains the usual stabilizing agents as described for commercially available urokinase can contain about 50,000-200,000 IU of uPA per unit.

An example of a lyophilized pharmaceutical formulation for reconstitution in 2 ml sterile saline for e.v. injection is the following:

3F11 scu-PA     5 mg
mannitol     20 mg
sodium edetate     2 mg
phosphate buffer q.s.

A more detailed explanation of an embodiment of the present invention relating to the preparation of the transfection vectors and means is given in the following paragraphs (from A.1 to A.7)

A.1 - Cloning of the human uPA gene

A human genomic DNA bank is obtained cloning partially digested liver DNA in the Charon 28 lambda vector. uPA gene containing phages are rescued by screening the genomic bank with a suitable probe such as a probe obtained from a partially unspliced human uPA cDNA. The positive phage clones are isolated, assayed by restriction analysis to isolate those which contain the uPA open reading frame (ORF) or the 5' region that contains the uPA promoter.

A.2 - Preparation of uPA coding DNA

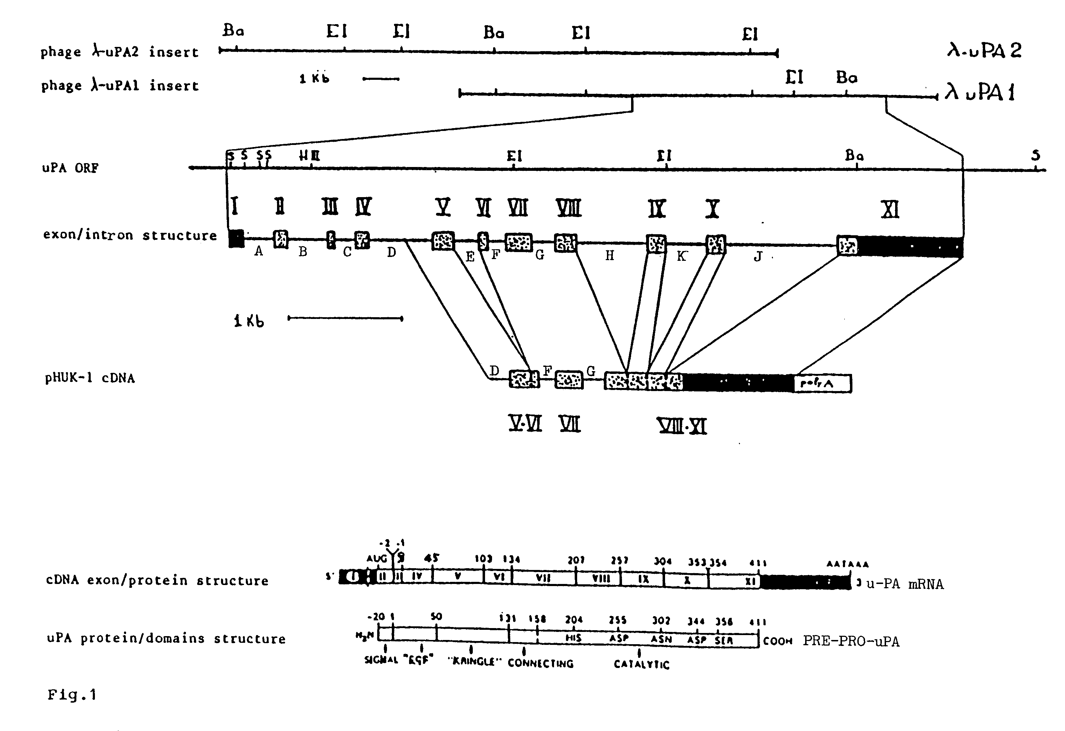

Close to the 5' end of the human uPA mRNA there are four SmaI restriction sites and the closest rightward lies outside the coding region, beyond the uPA polyadenylation site. The 5' sites are clustered in a short segment of DNA within the first exon and the first intron in a region preceeding the translational initiation codon located at the beginning of the second exon (Fig. 1)
Restriction analysis of the lambda-uPA1 phage clone revealed that, at least under our digestion conditions, only the first and the fifth SmaI sites are usually cleaved. Therefore, by SmaI digestion of a lambda-uPA1 and DNA fragment separation on agarose gel, it possible to cut out the uPA coding region and subsequently insert it into a different vector. By doing so, another SmaI fragment is also obtained, more precisely, 2.38kb SmaI fragment that lies 5' to the uPA ORF and contains the uPA mRNA starting site, promoter and regions regulating it through trans-regulating factors. This fragment can regulate the mRNA trascription of a 3' adjacent gene.

A.3 - Expression of the uPA gene in animal cells

In order to express the human uPA gene in mammalian cells the DNA region coding for uPA mRNA must be placed under the trascriptional control of a eukaryotic promoter. A number of different signals (e.g. enhancer and polyadenilation sites) may influence the rate of mRNA trascription and the mRNA stability (Y. Gluzman, Viral vectors, Cold Spring Harbor Press, New York).

A.4 - Construction of RSV derived vectors

An example of a eukaryotic promoter that can be suitably employed in the process of the present invention is the long terminal repeat (LTR) of the Rous Sarcoma Virus (RSV) (see D.E. Schwartz et al, Nucleotide sequence of Rous Sarcoma Virus, Cell 32, 853 (1983)). A preferred RSV derived plasmid is the "plasmid RSV-Neo" wherein the gene for the resistence of the antibiotic neomycin, cloned from the bacterial transposon Tn5, is inserted in the RSV LTR downstream to the RSV enhancer and promoter and 5' to the SV 40 polyadenilation site. Since it contains also the pBR322 derived ampicillin resistance and origin of replication functions, the RSV-Neo plasmid can be propagated in E. coli. After transfection into mammalian cells, RSV-Neo confers to these cells the resistance to neomycin and its analogues (see F. Colbere-Garapin et al., A new dominant selective marker for higher eukaryotic cells; J. Mol. Biol. 150, 1 (1981) and P.J. Southern and P. Berg, Transformation of mammalian cells to antibiotic resistance with a bacterial gene under the control of the SV40 early region promoter; J. Molec. and Appl. Genetics, 1, 327 (1982)). A vector for expressig human uPA may be obtained from RSV-Neo by substituting the neomycin resistance gene which is between the RSV promoter and the SV 40 polyadenilation site with the uPA coding region. Thus, using RSV-Neo as a starting material, the human uPA gene is inserted in the RSV LTR in the same transcription orientation; the plasmid so obtained is called RSV-sc-uPA. Transformation of mammalian cells with this plasmid confers them the ability to secrete uPA in the culture medium.
Briefly, an uPA expressing vector (RSV-uPA) can be constructed as follows:
a) a 7.3 kb fragment containing the uPA coding region is obtained from the insert of the phage lambda-uPA1;
b) a DNA fragment containing the LTR of the RSV, the SV 40 polyadenylation site and the pBR322 derived ampicillin resistance and prokaryotic origin of the replication is obtained from the plasmid RSV-Neo;
c) the uPA coding region is inserted under the control of the RSV promoter creating the bacterial plasmid RSV-uPA;
d) RSV-uPA is co-transfected in mammalian cells together with RSV-Neo and

e) antibiotic resistant, uPA producing clones are selected and further propagated.

A.4.1 - Preparation of vector DNA

RSV-Neo is a known and currently available plasmid that contains the neomycin resistance gene from the bacterial transposon Tn5 under the trascriptional control of the RSV promoter. When transfected in mammalian cells, the plasmid confers them the resistance to the neomycin analogue G418.

RSV-Neo is digested with HindIII, the 5' protruding ends are filled in with the Klenow fragment of DNA polymerasel, digested with Hpal and treated with Calf Intestine Phosphatase (CIP). These treatments generate two blunt-ended, dephosphorylated fragments, the smallest of which contains the origin of replication and the ampicillin resistance gene coming from plasmid pBR322 and has, at its extremities, the RSV promoter and the SV 40 polyadenylation site (PAS). After separation on agarose this 3.5 Kb fragment is purified and used as a eukaryotic expression frame in which the uPA gene can be inserted.

A.4.2 - Cloning of the uPA gene under the RSV promoter

Insert and vector DNAs are ligated together and used to transform competent E.coli cells. Among the ampicillin resistant colonies, RSV-uPA is selected which has the human uPA coding region inserted in the appropriate orientation in the RSV LTR.

A.5 - Construction of BPV derived episomic vectors

A possibility of increasing the protein production of mammalian cells transformed with foreign genes is to increase the gene copy number, e.g. using dehydrofolate reductase (DHFR)-based vectors or Bovine Papilloma Virus (BPV)-derived vectors.

BPV is a 7945bp long, double stranded, circular DNA virus naturally occurring in cattle papillomas (see C.Y. Chen et al., The primary structure and the genetic organization of the Bovine Papilloma Virus type 1 genome, Nature 299, 529 (1982)). Infected cells maintain the virus DNA extrachromosomally as a stable episome at a high copy number, (see M.F. Law et al, Mouse cells transformed by Bovine Papilloma Virus contains only extrachromosomal viral DNA sequences, Proc. Natl. Acad. Sci. USA 78, 2727 (1981), and W.D. Lancaster, Apparent lack of integration of bovine papilloma virus DNA in virus-induced equine and bovine tumor cells and virus-transformed mouse cells, Virology 108, 251 (1981)). The BPV genome can be roughly subdivied into three functional regions: a non-coding, regulatory region about 1kb long, between the HindIII and Hpal sites, a transforming region spanning approximately 60% of the genome between Hpal and BamHI and a region coding for the virus structural proteins from BamHI to HindIII (see M. Lusky and M. Botchan, Characterization of the Bovine Papilloma Virus plasmid maintenance sequences, Cell 36, 391 (1984), and B.A. Spalholz et al, Transactivation of a Bovine Papilloma Virus transcriptional regulatory element by the E2 gene product, Cell 42, 183 (1985)). BPV DNA can be used to transform rodent cells but BPV particles are not produced in in vitro transformed cells because these do not allow expression of the BamHI-HindIII region (E. Antmann, BPV trascription, polyadenilated RNA species and assesment of the direction of trascription, J. Virol. 43, 59 (1982), and L.W. Engel, Trascription organization of the BPV type, J. Virol. 47, 516 (1983)). However, this region does not seem to be necessary for cell transformation. Hybrid plasmids have been constructed using BPV and pMLd, which is a pBR322 derived plasmid wherein some "poison" sequences inhibiting the virus replication in mammalian cells have been deleted (M. Lusky and M. Botchan, Characterization of the Bovine Papilloma Virus plasmid maintenance sequences, Cell 36, 391 (1984); B.A. Spalholz et al, Transactivation of a Bovine Papilloma Virus transcriptional regulatory element by the E2 gene product, Cell 42, 183 (1985)). These recombinant viruses can be propagated in E. Coli as well as in animal cells and easily used in recombinant DNA procedures (D. DiMaio et al., BPV vector that propagates as a plasmid in both mouse and bacterial cells, Proc. Natl. Acad. Sci. USA 79, 4030 (1982); P.J. Kushner et al, A plasmid that replicates in both mouse cells and E. coli , J. Mol. Appl. Gen. 1, 527 (1982), N. Sarver et al, BPV deoxyribonucleic acid: a novel eukaryotic cloning vector, Molec. and Cell. Biol. 1, 486 (1981)). The ability of the BPV genome to transform in vitro rodent cells, is maintained in the hybrid plasmids (C.A. Heilman; Virus specific trascription in BPV transformed mouse cells; Virology 119, 22 (1982)) that may therefore be used as mammalian expression vectors (F. Colbere-Garapin et al, A new dominant selective marker for higher eukaryotic cells, J. Mol. Biol. 150, 1 (1981)); P.J. Southern and P. Berg, Transformation of mammalian cells to antibiotic resistance with a bacterial gene under the control of the SV40 early region promoter, J. Molec. and Appl. Genetics, 1, 327 (1982); N. Sarver et al., Transformation and replication in mouse cells of a BPV-pML2 plasmid vector that can be rescued in bacteria, Proc. Natl. Acad. Sci. USA 79, 7147 (1982); N. Sarver et al, BPV shuttle vectors, Gene expression, J. Setlow and A. Hollander ed, 173, Plenum Press, New York; G. Meneguzzi et al, Plasmidial maintenance in rodent fibroblasts of a BPV1-pBR322 shuttle vector without immediately apparent oncogenic transformation of the recipient cells, EMBO J. 3, 365 (1984)). Foreign DNA may block BPV functions (e.g. plasmidial maintenance, copy number regulation, BPV DNA replication and cell transformation) when inserted at positions different from the boundaries of the three regions. Expression of foreign genes and cDNAs has been shown to be at least partially dependent on the site of insertion and the sense of trascription (N. Sarver et al, Enhancer-dependent expression of the rat preproinsulin gene in BPV type 1 vectors Mol. Cell. Biol. 5, 3507 (1985)). A number of foreign genes have been inserted in recombinant plasmids and used to express genes placed under the trascriptional control of constitutive and inducible promoters: Y. Gluzman, Viral vectors, Cold Spring Harbor Press, New York, P.J. Kushner et al, A plasmid that replicates in both mouse and E. coli cells J. Mol. Appl. Gen. 1, 527 (1982); N. Sarver et al, BPV shuttle vectors, Gene expression, J. Setlow and A. Hollander ed, 173, Plenum Press, New York; N. Sarver et al, Enhancer-dependent expression of the rat preproinsulin gene in BPV type 1 vectors, Mol. Cell. Biol. 5, 3507 (1985); E.T. Scherborn et al, Expression of a human U1 RNA gene introduced into mouse cells via BPV vectors, Mol. Cell. Biol. 5, 1318 (1985); M. Eiden et al, Type 1 Human T-cell Type Leukemia Virus small envelope proteins expressed in mouse cells by using a BPV-derived shuttle vector, Mol. Cell. Biol. 5, 3320 (1985); P.M. Howley et al, Eukaryotic cloning vectors derived from BPV DNA; Methods in enzymology 101, 387 (1983), D. DiMaio et al, High level expression of a cloned HLA heavy chain gene introduced into mouse cells on a BPV vector; Mol. Cell. Biol. 4, 340 (1984); N. Husiung et al, Efficient production of Hepatitis B surface antigen using a BPV-Metallothionein vector, J. Molec. Appl. Genet.; Y. Wang et al, Enhanced production of Hepatitis B surface antigen in NIH3T3 mouse fibroblasts by using an extrachromosomally replicating BPV vector, Mol. Cell. Biol., 3, 1032 (1983); M. Karin et al, Expression and regulation of a human metallothionein gene carried on an autonomously replicating shuttle vector, Proc. Natl. Acad. Sci. USA 80, 4040 (1983); L. Maroteaux et al., Cyclohexamide induces expression of the human interferon beta 1 gene in mouse cells transformed by BPV-interferon beta-1 recombinants, J. Virol. 47, 89 (1983); S. Mitrani-Rosenbaum et al., Inducible expression of the human interferon beta-1 gene linked to a BPV DNA vector and maintained extrachromosomally in mouse cells, Mol. Cell. Biol. 3, 233 (1983): G.N. Pavlakis and D.H. Hammer, Expression of cloned growth hormone and metallothionein genes in heterologous cells, Recent Progress in Hormone Research 39, 363 (1983). The BPV 230.8 plasmid contains the BPV DNA opened at the BamHI site and cloned at the same site of the pBR322-derived pML2d plasmid (see B. Binetruy et al, Recombinant DNA molecules comprising BPV type 1 DNA linked to a plasmid DNA are maintained in a plasmidial state both in rodent fibroblasts and in bacterial cells, EMBO J. 1, 621 (1982)).

Episomic expression vectors have manily two advantages:

a) differently from transformations in wich the transfected DNA is inserted in the cell chromosomal DNA, episomic vectors mantain the gene to be expressed in a defined enviroment;

b) the gene copy number is usually higher and more stable than that obtainable with inserted genes.

A series of BPV derived vectors containing the RSV promoter-uPA ORF trascription unit is constructed as follows:

I) a unique XhoI site is inserted in pBPV230.8 at three defined different positions obtaining three different vectors;

II) RSV-uPA is modified inserting two XhoI sites at the ends of the RSV-uPA trascription unit;

III) the RSV-uPA trascription unit is cut out with XhoI and inserted in the three BPV derived vectors in both orientations;

IV) the six plasmids are tested for their ability to produce uPA co-transfecting them together with RSV-Neo in mammalian cells and then selecting antibiotic-resistant, uPA-producing clones.

A.5.1 - Restriction site modification of BPV230.8

To avoid the risks of unfavorable site of insertion or gene orientation (e.g. promoter dampening), it is advisable to insert the RSV-uPA construction at three different sites of the BPV230.8 plasmids in both orientations. To do this, the plasmids restriction sites BamHI, HindIII and SalI are modified in separate experiments by insertion of a phosphorilated XhoI linker. Plasmid DNA is first digested with the appropriate enzyme, then the protruding ends are filled in with the Klenow fragment of DNA PolymeraseI, an XhoI linker is ligated in large excess, the obtained DNA fragment is digested with XhoI, self-ligated and used to transform E.coli competent cells.

Three plasmids are obtained:

- pBB, in wich the new unique XhoI site is inserted at the BamHI position and is flanked by two BamHI sites created by the linker/vector fusion;
- pBS, in wich the new unique XhoI site is inserted at the SalI position and is flanked by two SalI sites created by the linker/vector fusion;
- pBH, in wich the new unique XhoI site is inserted at the HindIII position.

## A.5.2 - Restriction site modification of RSV uPA

In the RSV-uPA plasmid the restriction endonucleases NdeI and ApaI cut only once each, the first in the pBR322 derived region close to the RSV promoter and the second in the SV 40 region 3' to the uPA polyadenilation site. These two sites can be modified by XhoI linkering as follows:

the plasmid DNA is double digested with NdeI and ApaI, the protruding ends are blunted and modified with a XhoI linker. The fragment containing the origin of replication and the ampicillin resistance is dephosphorylated and then ligated to the fragment containing the RSV/uPA expression functions. A plasmid is obtained, in wich the RSV/uPA construction is flanked by XhoI sites, which is named plasmid p71. After XhoI digestion, it gives a DNA fragment containing the RSV promoter/uPA coding region suitable to be cloned in any vector having a XhoI or SalI site or which may appropriately be modified to have these insertion sites.

## A.5.3 - Insertion of RSV/uPA in BPV derived vectors

In separate experiments pBB and pBH are digested with XhoI while pBS is digested with SalI and the linearized vectors are dephosphorylated with CIP. The fragment containing the RSV/uPA construction is obtained by restricting plasmid p71 with XhoI and separating the two resulting fragments on agarose gel. Vector and insert DNAs are ligated together, digested with XbaI that cuts only once in BPV, then self-annealed and used to transform E.coli competent cells. A certain number of plasmids results which carry the RSV/uPA trascription construction in both orientation at the 3 different positions.

## A.6 - Preparation of the human uPA promoter

Eukaryotic genes that are transcribed by RNA polymerase II have been shown to be under the transcriptional control of very complex promoters. Activation and/or repression of these promoters lead to the cell cycle phase, tissue and differentiation stage specific expression of each gene. Moreover, extracellular factors (e.g. hormons, ions, temperature) can activate, modulate or repress the steady-state level of activity through very different mechanisms. DNA binding factors, the nuclear matrix and covalent modifications of the DNA, such as methylation, gives the promoter the ability of transcribe the nearby gene.

A DNA fragment located immediately upstream of the open reading frame (ORF) coding for the proUK and shown to contain the proUK mRNA starting site and promoter is cloned in the SmaI site of the promoterless plasmid pEMBL8-CAT upstream to the bacterial chloramphenicol acetyl transferase (CAT) reporter gene. The resulting plasmid (named puPA2) is modified in the SmaI site 5' to the promoter by inserting a XhoI linker to facilitate further modifications. The puPA2 plasmid and its derivative are eventually transfected in cultured mammalian cells and their ability to drive trascription tested evaluating the CAT activity.

The results of this test indicate that the uPA promoter can drive the CAT gene trascription at levels comparable to those given by the RSV promoter. Therefore the puPA2 plasmid and its derivative are powerful expression vectors when a foreign gene or cDNA is inserted in the polylinker region downstream to the uPA promoter. Otherwise a plasmid fragment containig the uPA promoter region can be used as an "expression cassette" in front of genes and cDNAs by inserting them into the plasmid polylinker (which is derived from pEMBL8).

A.6.1 - uPA expression vectors

Digestion of lambda-uPA1 DNA with SmaI produces, among the others, a fragment containing the human uPA promoter (see A above). This fragment can be cloned upstream from a gene to drive its trascription in suitable mammalian cells. Such construction can confer mainly three advantages:
- the expression vector does not contain any sequences of viral origin;
- the promoter may contain sequences that confer it the property to be induced by signals such as ions, chemicals and hormons;
- promoter derived sequences retained in the 5' end of the mRNA may confer it higher stability.

Briefly, vectors in which the human uPA promoter may drive the transcription of 3' adjoining gene are prepared as follows:

a) a 2.38kb fragment is obtained from lambda-uPA1 by SmaI digestion;

b) this fragment is inserted in the proper orientation in a plasmid such as the plasmid pEMBL8 CAT at the SmaI site thus giving the plasmid puPA2;

c) the SmaI site 5' to the promoter is converted into a XhoI site by linkering, thus obtaining a plasmid named puPA2/41 (this plasmid, like puPA2, can express the adjacent CAT gene when inserted in a suitable mammalian cell);

d) the CAT gene is excised by partially digesting puPA2/41 with XbaI, filling in the protruding ends, ligating a SalI linker and digesting with SalI obtaining the plasmid pPP41;

e) a 7.3kb SmaI fragment is obtained from lambda-uPA1 phage which contains the uPA ORF (see A.2) and inserted in the proper orientation in the unique SmaI site of pPP41 to give the uPA expression vector named pPP41XS;

f) pPP41XS is co-transfected with RSV-Neo and

g) antibiotic resistant, uPA producing clones are selected and propagated.

A.6.2 - Construction of the puPA2 plasmid

To obtain the puPA2 plasmid, DNA from the lambda-uPA1 phage is digested with SmaI. A band of 2.38 kb is gel purified and inserted in the pEMBL8 vector linearized with the same enzyme and dephosphorylated with CIP. Bacterial colonies carrying the recombinant plasmid having the promoter in the correct orientation are selected. One of these, named puPA2, contains the 2.38 kb fragment of the prouPA promoter 5' to the CAT gene and drives the transcription of the bacterial gene when transfected in prouPA producing cells.

A.6.3 - Construction of the puPA2/41 plasmid

To insert a unique site at the 5' end of the pro-uPA promoter that can be used to further modify the puPA2 plasmid, it can be partially digested with SmaI and the restriction site modified by insertion of an XhoI linker. The resulting plasmid, named puPA2/41, has the SmaI site at the upstream end of the uPA promoter substituted by a unique XhoI site.

The ability of the puPA2 plasmid and that of its derivatives to drive the trascription of the adjoining CAT gene is compared to that of RSV-CAT, a plasmid in wich the CAT gene is under the trascriptional control of the RSV LTR. The plasmids are introduced in A 1251 cells (a human cell line derived from a kidney carcinoma) by calcium phosphate precipitation and the CAT activity is measured in a transient expression assay.

puPA2/41 DNA is then partially digested with XbaI, the protruding ends are filled in with the Klenow fragment of DNA PolI, ligated to an excess of SalI linker and digested with SalI. A fragment of 4.8kb is purified on agarose gel, self-ligated and used to transform E.coli competent cells. A plasmid (named pPP41) is selected by miniprep analysis; a foreign gene can be inserted in the pEMBL8 derived polylinker downstream the uPA promoter and the uPA mRNA trascription start point. The promoter gene costruction can be excided from pPP41 using XhoI and SalI and inserted into a XhoI or SalI site of a given plasmid.

## A.6.4 - Insertion of the uPA gene in pPP41

The uPA ORF containing 7.3kb fragment obtained by SmaI digestion of lambda-uPA1 DNA is ligated in the SmaI digested, CIP treated pPP41 DNA and used to transform E.Coli competent cells. Among the ampicillin resistent colonies, a plamid (named plasmid pPP41XS) is selected which carries the uPA ORF under the control of its own promoter and a region adjcent thereto. This construction re-establish the original SmaI site downstream the mRNA start point and the original mRNA trascription sequence.

## A.7 - Construction of uPA promoter deletion derivatives

Eukariotic promoter modifications, such as deletions, insertions or mutations can confer to a promoter new characteristics such as a broader specificity and a higher transcription level. The fact that inducible regions may still be conserved in such promoters and thus can be modulated by external factors renders them particularly useful when the produced protein is such that it would negatively interfere with the biology of the cell.

Deletions internal to a 2.38kb DNA fragment derived from the human uPA promoter can be conveniently obtained by genetically engeneering the bacterial plasmid puPA2/41 described above. Preferably, deletions are made by taking advantage of the restriction sites which are already present in the uPA promoter. Two plasmids are obtained with this procedure:

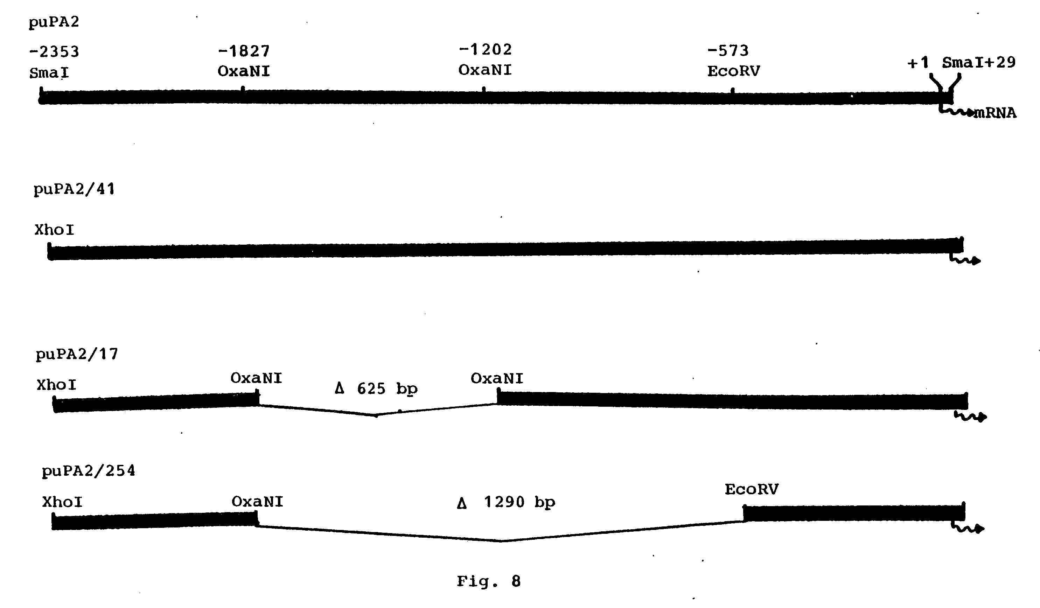

- a plasmid, named puPA2/17, which has a 625bp deletion spanning the two OxaNI sites;
- another plasmid, named puPA2/254, which has a 1.28kb deletion spanning from the OxaNI site at position 5080 to the EcoRV site at position 6369 (referred to the puPA2 plasmid map, see Fig 8).

These two plasmids carry the bacterial CAT gene downstream to the modified uPA promoter and therefore the promoter activity can be easily detected by measuring the CAT activity after transfection of the plasmid in eukaryotic cells.

A different gene can be easily inserted under the control of these deletion promoters taking advantage of the polylinker caming from the pEMBL8 frame or these deletion promoters can be used as "expression cassettes" containing, 5′ to 3′, the promoter, the mRNA starting site and a polylinker and inserted upstream of a gene contained in a different vector.

Using this construction we expressed also the uPA gene. Briefly, Constructions are made as follows:

a) puPA2/41 is completely digested with OxaNI obtaining puPA2/17;

b) puPA2/41 was completely digested with EcoRV, OxaNI and filled in with the Klenow fragment of DNA PolI obtaining the plasmid puPA2/254;

c) the two uPA-derived deletion promoters are tested for their ability to drive the trascription in a CAT essay;

d) the CAT gene is excised from both puPA2/17 and puPA2/254 as described above (A6.3);

e) the uPA ORF is inserted under the control of the two deletions promoters;

f) the two uPA expression vectors are transfected in mammalian cells.

## BRIEF DESCRIPTION OF THE DRAWINGS

The accompanying drawings are intended to further illustrate the invention and the reference to the drawings in the description and claims is mainly by way of illustration and is not intended to be a limitation to the scope of the present invention. The following brief description of the drawings is intended to make the understanding of the drawings and of the invention as a whole easier.

## Fig.1 Structure of the uPA gene, mRNA and protein

From top to the bottom:
- restriction map of the uPA gene insert in phage lambda-uPA2;
- restriction map of the uPA gene insert in phage lambda-uPA1;
- restriction map of the uPA "open reading frame" (Ba: BamHI; EI: EcoRI; S: SmaI; HIII: HindIII);
- exon/intron structure of the human uPA gene: black boxes: untranslated 5′ and 3′ regions; shadowed boxes: translated exons; lines: introns;
- exon/intron structure of the partially unspliced cDNA clone pHUK1;
- correspondence between exons and aminoacids in the pro-uPA mRNA;
- correspondence between protein domains and amino acids in pro-uPA.

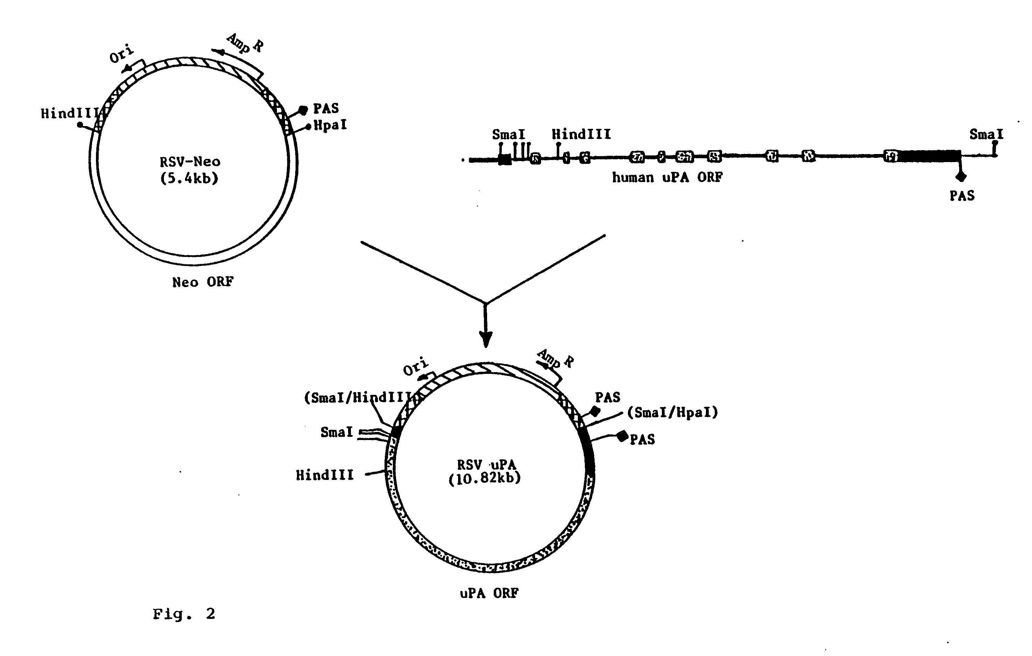

### Fig.2 Costruction of RSV-uPA

A SmaI DNA fragment containing the complete ORF for the uPA mRNA obtained from lambda-uPA1 (see the top portion of this figure) is inserted in the sense of the transcription in the HindIII and HpaI digested RSV-Neo plasmid which carries the bacterial plasmid functions and the LTR of the RSV (see the left portion of this Figure). RSV-uPA has the uPA ORF under the control of the promoter contained in the RSV LTR and can express the protein when inserted in a suitable eukaryotic host (see the bottom portion of this Figure).

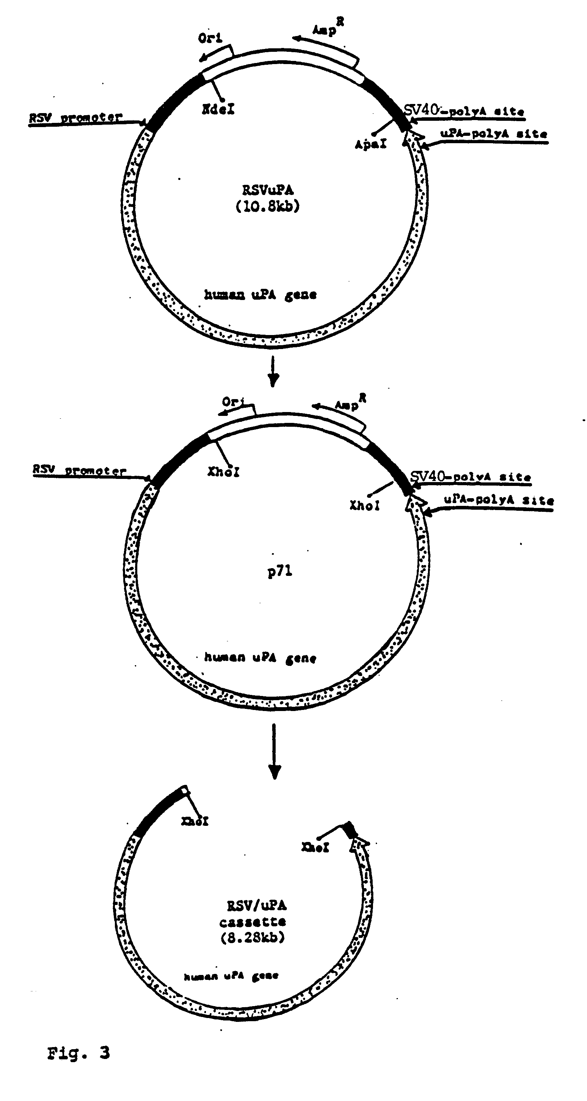

### Fig.3 Restriction site modification of RSV-uPA

The RSV-uPA sites NdeI and ApaI are replaced by two XhoI sites by linkers insertion generating the plasmid p71 (Fig. 3 in the middle). Digestion of the p71 plasmid with XhoI gives a fragment containing the uPA ORF under the control of the RSV promoter that can be inserted either in XhoI or SalI containing vectors (Fig. 3 below).

### Fig.4 Restriction map of pBPV-230.8

pBR322 derivative pML2d is inserted at the unique BamHI site of the BPV-1 genome (Saver et al. Mol. Cell. Biol. 5, 3507; 1985) and the BamHI site adjacent to the 3′ end of the transforming region is modified to SalI.

### Fig.5 Insertion of the RSV-uPA construct in episomal vectors

Plasmid pBPV230.8 is modified by replacing one of the three viral sites BamHI, HindIII and SalI obtaining respectively pBB, pBH and pBS. The new XhoI site is boxed in each derived vector. The new BamHI and SalI sites resulting from the insertion of the XhoI linker and flanking the XhoI site in pBB and pBS are showed. In the three plasmids the RSV/uPA construct (see Fig. 3) is inserted in the XhoI site in both orientations obtaining the following plasmids:
- pBBuPAa, pBHuPAa and pBSuPAa wherein uPA and BPV mRNAs are transcribed in the same sense of BPV;
- pBBuPAb, pBHuPAb and pBSuPAb, wherein uPA mRNA and the BPV mRNAs are transcribed in opposite direction.

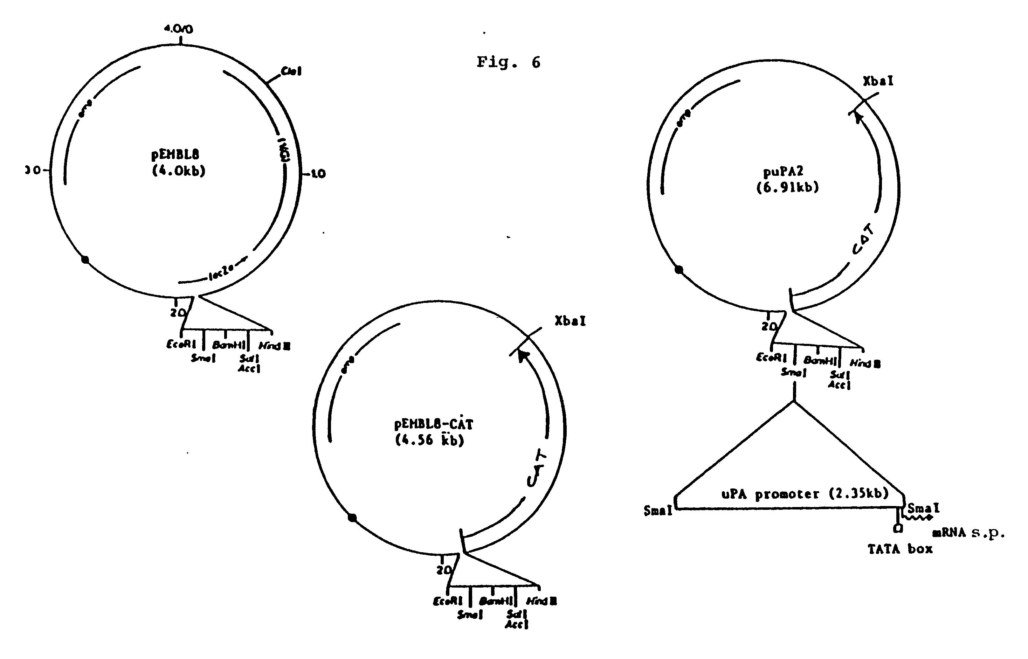

### Fig.6 Construction of puPA2

Plasmid pEMBL8-CAT is derived from pEMBL8 by substituting the fragment between ClaI and HindIII with a fragment carrying the bacterial CAT gene (Dente et al., Nucl. Acid Res., 1645-1655; 1983). A SmaI fragment containing the uPA promoter is inserted upstream from the CAT gene generating the plasmid puPA2 (this figure, on the right) having the correct promoter sequence orientation to transcribe the CAT gene.

12

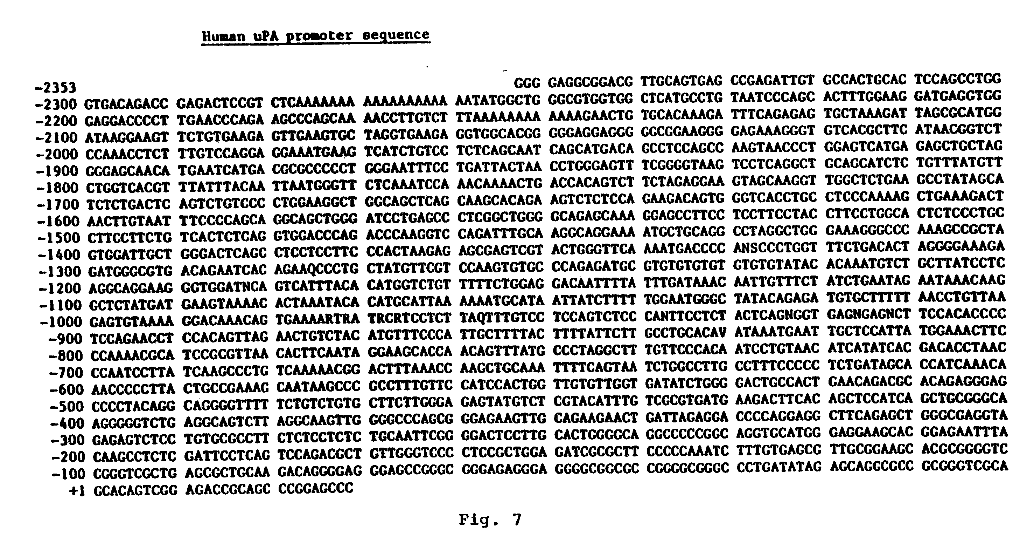

Fig.7 - Sequence of the human uPA promoter

The sequence of a 2.38kb SmaI fragment from lambda-uPA1 phage DNA, containing the mRNA start point, the TATA box and the upstream regulatory regions of the uPA promoter, is shown. Nucleotide +1 on the map corresponds to the first nucleotide in the uPA mRNA described by Riccio et al. using primer extension analysis.

Fig. 8 Restriction map of the uPA promoter and its derivatives

The restriction map of the puPA2 modified plasmids indicating where the uPA promoter has been modified is represented; in particular:

- puPA2/41, wherein the SmaI site at the 5′ end of the promoter is converted to XhoI;
- puPA2/17, wherein the region between the two OxaNI sites has been deleted;
- puPA2/254, wherein the region between the 5′ OxaNI site and the EcoRV site has been deleted.

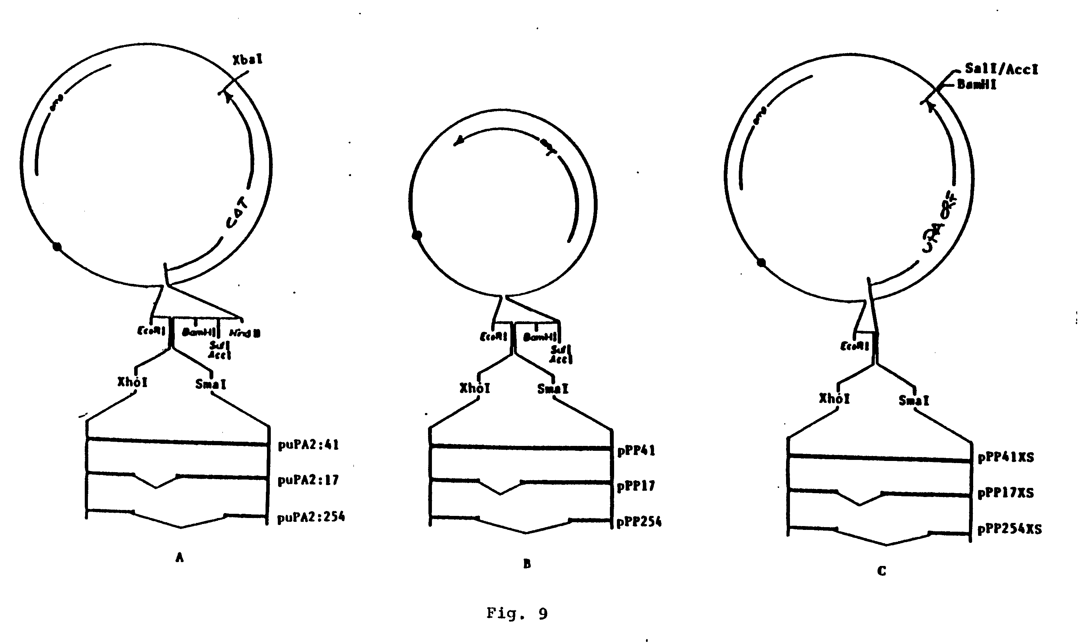

Fig. 9 uPA promoter derived vectors

Fig. 9A depicts the restriction map of plasmids puPA2/41, puPA2/17 and puPA2/254

Fig. 9B depicts the restriction map of plasmids pPP41, pPP17 and pPP254 that are obtained from puPA2/41, puPA2/17 and puPA/254, respectively, by excising the CAT gene by SalI/XbaI digestion and SalI linkering.

These vectors contain the uPA promoter (or a derivative thereof), the TATA box, and the mRNA start point and can be used to express genes or cDNAs by inserting them, for example, at the blunt-ended SmaI site or at the BamHI site with fragments having MboI, BglII, BclI and BamHI ends. These constructs can be excised by double digesting with XhoI and SalI. The same double digestion can be used to excise the promoter construct to insert it, as an "expression cassette", in a different plasmid 5′ to the gene or cDNA to be expressed. The filled circle indicates the plasmid origin of replication.

Fig. 9C depicts plasmids pPP41XS, pPP17XS, and pPP254XS, which are derived from pPP41, pPP17 and pPP254, respectively, by inserting a 7.3 kb SmaI fragment containing the uPA ORF in the plasmid SmaI site.

Fig. 10

N-terminal aminoacid sequence (18 residues)
of 3F11 scu-PA in comparison with human kidney scu-PA (Kohno T., Hopper P., Lillquist J.S., Suddith R.L. Greenlee R., Moir D.T., Biotechnol. 1984, 2, 628-634) Human A431 scu-PA, human urine scu-PA, and mouse scu-PA.

Fig. 11

SDS-PAGE (sodium dodecyl sulfate polyacrylamide gel electrophoresis) of:
a) scu-PA of urinary origin
b) scu-PA from A431 cells
c) RSV-A431-scu-PA
d) RSV-LB6-scu-PA
e) BPV-LB6-scu-PA
f) RSV-CHO-scu-PA in non-reducing conditions.
The rightmost lane shows molecular weight standards.

Fig. 12

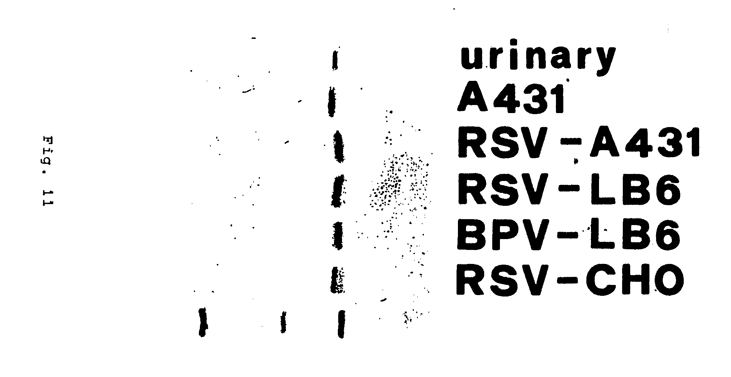

SDS-PAGE of the scu-PA's reported in Fig. 11 (in the same order) after activation with plasmin to transform them into the corresponding tcu-PA's, under reducing conditions (see 1.6.2). The rightmost lane shows molecular weight standards.


Fig. 13

SDS-PAGE comparison of tcu-PA's obtained by activation with plasmin of the scu-PA's reported in Fig. 11 (in the same order) untreated and after treatment with glycopeptidase F, under reducing conditions (see 1.6.5). A 1:1 mixture of untreated tcu-PA and glycopeptidase F treated tcu-PA was loaded on each lane. The rightmost lane shows molecular weight standards.


Fig. 14

SDS-PAGE comparison of tcu-PA's obtained by activation with plasmin of the scu-PA's reported in Fig. 11 (in the same order) untreated and after treatment with neuraminidase under reducing conditions (see 1.6.5). A 1:1 mixture of untreated tcu-PA and neuraminidase treated tcu-PA was loaded on each lane. The leftmost lane shows molecular weight standards.


## EXAMPLES

The following examples are intended only as an illustration of the invention and of the way in which it can be exploited, but cannot be construed as a limitation upon its scope. To make their understanding easier, we report in the paragraphs preceeding them (from 1.1. to 1.6.7) the techniques and methods (such as enzyme modification, DNA precipitation, protein purification, etc.) which are applied, essentially in the same manner, in different examples. In the examples, in fact, instead of repeating the whole procedure, the paragraph where it is described is oftentimes referred to.

1.1. The cell used are commercially available or otherwise available through the scientific community.

1.2. The cells are grown in an appropriate medium such as Dulbecco's modified Eagle medium (DMEM) containing 10% heat-inactivated fetal calf serum.

1.3 - Media, buffers and solutions for molecular biology.

When available, reagents are "DNA/RNA grade" and "analytical grade" in all the other cases. If not differently indicated, liquid and solid media for bacteria and phage growth, buffers and solutions are prepared, stored and used as as indicated in Maniatis T., Fritsch E.F., Sambrook J., Molecular Cloning, a laboratory manual, Cold Spring Harbor Laboratory, (1982), herein "Maniatis et al". If not differently indicated, glassware, plasticware and laboratory hardware are prepared, stored and used as indicated in the same reference. Deionized/distilled water is used, autoclaved if to be used in enzyme buffers.


1.4 - Enzymes for molecular biology

Restriction endonuclease, DNA modyfing enzymes and RNase are commercially available. We used those purchased from Boehringer, Bethesda Research Laboratories (BRL), Collaborative Research, New England Biolabs and Pharmacia; lysozyme and bovine serum albumine (BSA) are also commercially available. We purchased lysozyme from Sigma and BSA from Boehringer. Commonly used buffer solutions are used for each enzyme reaction. Their composition and preparation are generally known and conveniently they are prepared according to the information accompanying each enzyme, the preparations are filter sterilized and stored in aliquotes at -20° C. Many buffers of current use are also commercially available as concentrates. Synthetic DNA linkers are commonly available as well as t-RNA and herring sperm DNA. We purchased synthetic DNA linkers from P-L Pharmacia or New England Biolabs, while tRNA and herring sperm DNA were from Boehringer.

### 1.5 - rDNA tecniques

If not differently indicated in the following examples, rDNA techniques are performed as described in Maniatis et al. and in the original references therein contained.

### 1.5.1 - Restriction digestion and DNA enzymatic modification.

Single and multiple restriction digestions and DNA enzymatic modifications are performed as known in the art, and also reported in commonly available manufacturers' booklets and in Maniatis et al, ch.4. "Heat inactivation" means inactivating the enzyme at 70° C for 10-15 min, then leaving the mixture from 10 to 15 min at room temperature and spinning down 2 min in an Eppendorf centrifuge. Preferentially, restriction digestions and the results of enzymatic DNA modification are checked on 0.5 microg/ml ethidium bromide containing 1% agarose gels runned at 100 V for 1-2 h (see also Maniatis et al., ch.5).

### 1.5.2 - Phenol/chloroform extractions

Preferentially, phenol/chloroform extractions of proteins from nucleic acid aqueous solutions are performed as described in Maniatis et al., p. 458-459; DNA is "back-extracted" when the reaction volume is smaller then 200 microl.

### 1.5.3 - DNA precipitation

Preferentially, DNA precipitations are performed adding 0.1 vol. of 3M sodium acetate pH 5.2 and 2.5 volumes of ethanol (at -20° C). 20 to 40 microg of tRNA are added only when indicated in the following examples. The mixture is left in a dry ice/ethanol bath for 15 to 30 minutes or at -20° C overnight and then spinned down at 4° C for 15 min at 10000 x G in an Eppendorf microcentrifuge or in a Sorvall centrifuge. The DNA pellet is washed once with 70% ethanol and then once with 100% ethanol and finally dried in a Savant vacuum centrifuge.

### 1.5.4 - 5′ Dephosphorilation

Preferentially, 5′ DNA ends are dephosphorilated with commercially available calf intestine phosphatase (CIP, purchased from Boehringer), at 37° C for 1h at pH 8. CIP is removed by phenol:chloroform extraction, or by adding EGTA (ethylene bis (oxyethylenenitrilo) - tetracetic acid) to a final concentation of about 50mM and heating at 68° C for 45 min.

### 1.5.5 - DNA ligation

DNA ligations are performed as known in the art. Conveniently, T4 DNA ligase manufacturers' instructions are followed. Conveniently, ligase reactions are performed at 8° C when synthetic linkers are used, at 14 to 16° C in all the other cases. When blunt-ended DNA is to be ligated, PEG 1500 or PEG 4000 is added to a 10% final concentration. Conveniently, 10 x ligation buffer of the following composition is used: 0.66 M Tris-HCl pH 7.6, 50mM $MgCl_2$, 50 mM dithiothreitol, and 5mM ATP.

### 1.5.6 - DNA linkers

Preferentially, linker DNAs are used, phosphorylated and checked as indicated in Maniatis et al, pages 125-126, and used as indicated at page 392 and seq. Kinase reaction, ligation and restriction of the linker alone and of the linkered DNA fragments are checked by electrophoresis: the linker on a 2% agarose gel for 10 to 20 min (1 to 2 hours in the case of linkered DNA fragments) and then visualizing the DNA by autoradiography as indicated in Maniatis et al, page 172.

### 1.5.7 - Preparative gel electrophoresis

Preferentially, preparative gel electrophoresis is performed on 0.7% to 0.8% agarose gel containing 0.5 microg/ml ethidium bromide in 1X TBE buffer ( 0.089 M Tris, 0.089 M Boric acid and 0.002 M EDTA, Maniatis et al, pag. 156). The DNA is runned at 25V for the first 2 h and at 5OV for 16 to 20 h. DNA fragments are visualized on a 305nm UV transilluminator and DNA bands are cut out and electroeluted in an electroelution system (ISCO) in 0.1X TBE at 400V for 10 to 15 min. DNA is purified on Elutip-D column (Schleicher & Schuell) prepared and rinsed with a low salt buffer and eluted with a high salt buffer. Typically the column is prepared in a buffer containing 0.2 M NaCl, 20 mM Tris-HCl pH 7.3-7.5 (Tris is 2-amino-2-hydroxymethyl-1,3-propanediol) and 1.0 mM EDTA. This buffer is also used for rinsing, while the elution buffer contains 1.0 M NaCl, 20 mM Tris-HCl pH 7.3-7.5, and 1.0 mM EDTA. Finally the DNA is precipitated as indicated in 1.5.3.

### 1.5.8 - E. Coli transformation

Preferentially, E.Coli transformation (c.f. Hanahan D., Studies on transformation of E. Coli with plasmides, J. Mol. Biol. (1983) 155 557-580) are performed using commercially available or otherwise available competent cells such as strains DH1, DH5 or HB101 of BRL (Bethesda Research Laboratories) according to known per se techniques which are summarized also in the manufactures' booklet. Conveniently, the 100 microl (BRL) protocol is used when a DNA fragment is to be inserted in a different plasmid, while the small-scale, 20 microl (BRL) protocol is used when converting restriction sites by DNA linkers. After incubation without antibiotic, the cells are plated on agar LB plates (Maniatis et al. p.440-444) and incubated overnight at 37° C in the presence of the appropriate antibiotic.

### 1.5.9 - Screening of the transformed cells (miniprep analysis)

Preferentially, resistant colonies from DNA transformed competent E.Coli cells are screened by growing isolated colonies overnight at 37° C in 5ml of LB and the appropriate antibiotic (Maniatis et al., p. 440) and analyzing samples (1.5 ml) of the liquid culture with the boiling method as reported in Maniatis et al., p.366-367. Conveniently the following variations may be introduced:
- reaction volumes are scaled up 1.5 times;
- E.Coli strain DH1 is boiled for 60 sec;
- DNA precipitation is carried out without adding salt, but adding 1 vol of isopropanol and leaving the tubes at -20° C for 20 to 25 minutes;
- precipitated DNA is washed once with 1ml 70% ethanol and once with 1ml 100% ethanol;
- DNase-free RNase is added at 10 microg/ml and the DNA incubated 20 min at 70° C.

### 1.5.10 - Plasmid large scale preparation

Preferentially, plasmid large scale preparations are performed as described in Maniatis et al., ch.3. Preferentially the alkaline lysis method is used to recover the plasmid DNA (c.f. Maniatis et al, p.90-91), the plasmid DNA is purified twice on ethidium bromide containing CsCl gradients, then the mixture is extracted with isoamylic alchool, and the aqueous DNA containing phase is separated and dialized against TE pH8 (Maniatis et al.) and stored at 4° C.

### 1.6 - Purification and characterization of the obtained pro-urokinase.

### 1.6.1 - Chemicals:

all the chemicals used are either broadly commercially available or easily preparable from commercially available products. For example, the following list includes some chemicals used in the following examples and their sources: Affi-Gel 10 (activated agarose), acrylamide, bisacrylamide, sodium dodecyl sulfate, Comassie Brilliant Blu were from Bio Rad, Richmond, USA; CNBr-activated Sepharose, electrophoresis calibration kit for low molecular weight proteins were from Pharmacia, Uppsala, Sweden. Ampholine were from LKB AB, Bromma, Sweden; bovin serum albumin (BSA), Cohn fraction V, porcin trypsin, porcin plasmin, fibrinogen fraction I type IV, benzamidine, were from Sigma Chemical Co, St. Louis, MO 63178, USA; aprotinin was from Bayer, Leverkusen, GFR, glycopeptidase F from Boehringer Mannheim GmbH, FRG; S-2444 from Kabi AB, Stockolm, Sweden. High molecular weight urokinase (HMW-uPA) was produced by Lepetit Research Laboratories SpA Milano, Italy. Scu-PA (pro-uPA) can be prepared substantially following the procedure described by Stump D.C., Thienpoint M., Collen D., J. Biol. Chem. 261, 1267-1273, 1986. A431 scu-uPA is obtained according to the method described by Corti A., Nolli M.L., Soffientini A., Cassani G., in Thromb Haemostas 1986; 56, 219-224. Human endothelial plasminogen activator inhibitor (PAI-1) can be prepared substantially following the procedure described by van Mourik, J.A., Lawrence, D.A. and Loskutoff, D.J., Purification of an inhibitor of plasminogen activator (antiactivator) synthetized by endothelial cells, J. Biol. Chem. 259, 14914-14921, 1984.

All other reagents were analytical grade products commercially available from Merck Darmstad, GFR or Carlo Erba, Italy.

## 1.6.2 - Immunological and activity assays

The fibrinolytic assay is carried out by the fibrin plate method (Astrup T., Mullertz S., The fibrin plate method for estimating fibrinolytic activity, Arch Biochem Biophys 1952, 40:346-351). Urokinase reference standard is used to calibrate each assay. Activity is measured in International Units (I.U.) relative to the International Reference Preparation as the primary standard. sc-uPA (cell produced single chain urokinase) fibrinolytic latent activity is determined after activation as follows: plasmin (10 mg) is coupled with CNBr-activated Sepharose (cross-linked agarose) (2.5 ml) using 0.5 M sodium carbonate buffer as the coupling buffer. A 1:10 suspension of plasmin-agarose (0.9 ml) in 0.15 M sodium chloride, 0.05 M sodium phosphate buffer pH 7.4 is mixed with sc-uPA samples (0.1 ml) and gently rotated for 1 h at 20° C. Plasmin-agarose is removed by centrifugation 2 min at 10000 x g and the supernatant tested by the fibrin plate assay.

Protein concentrations were measured according to the method of Lowry (Lowry O., Rosebrough H., Parr A., Randall R., Protein measurement with the Folin phenol reagent, J. Biol. Chem. 1951, 193,265-275).

Amidolytic assays are performed by measuring the rate of hydrolysis of the synthetic substrate pyro-Glu-Gly-Arg-pNA, S-2444 (Claeson G., Friberger P., Knos M., Eriksson E., Methods for determination of pre-kallikrein in plasma, glandular kallikrein and urokinase, Haemostasis 1978; 7:76-78).

Immunoadsorbent-amidolytic assay (IAA) is carried out as described by Corti A., Nolli ML., Cassani G., Differential detection of single-chain and two-chain urokinase type plasminogen activator by a new immunoadsorbent amidolytic assay (IAA), Throm Haemostas 1986, 56:407-10. The assay combines the selectivity of immunoassays with the specificity of enzyme activity assays exploiting both the antigenic and enzymatic properties of the two proteins. tcu-PA and scu-PA are selectively immunoadsorbed on the wells of a microtiter plate coated with the monoclonal antibody 5B4 and tested for enzymatic activity before and after activation by plasmin treatment.

## 1.6.3 - Reverse phase FPLC

Reverse phase chromatography is carried out in a FPLC (Fast Protein Liquid Chromatography) system (Pharmacia Fine Chemicals) using a pro-RPC HR5/10 reverse phase chromatographic column (macroporous, microparticulated silica with bonded $C_1/C_8$ groups). Eluting buffers: buffer A, 0.1% trifluoroacetic acid in water, and buffer B, 0.1% trifluoroacetic acid in acetonitrile; elution mode: 100% A for 20 min, linear gradient from 0 to 60% B in A in 65 min, 100% B for 25 min; flow rate 0.3 ml/h.

## 1.6.4 - Electrophoretic procedures

SDS-polyacrylamide gel electrophoresis (SDS-PAGE) is carried out according to Laemmli UK, (Cleavage of structural proteins during the assembly of the head of bacteriophage T4, Nature 1970, 227:680-685), in gel slab 1.5 x 120 x 150 mm or in a fast, high resolution electrophoresis system (such as Phast System from Pharmacia, see also Olsson I. et al, Electrophoresis, 1988, 9, 16-22). The stacking and separating gels contained 3% and 12.5% acrylamide respectively. Marker proteins (Ampholines) of the commercially available electrophoresis calibration kit for molecular weight determination (Pharmacia) are run in each gel. 2-Mercaptoethanol is used as the reducing agent and Comassie Brilliant Blue R250 is used for gel staining. Immunoblotting is carried out essentially as known (c.f. Salerno G., Verde P., Nolli ML., Corti A., Szots H., Meo T., Johnson J., Bullok S., Cassani G., Blasi F., Monoclonal antibodies to human urokinase identify the single-chain pro-urokinase precursor, Proc Natl Acad Sci USA 1984, 81:110-114), using purified anti-uPA monoclonal antibodies such as Mab5B4 (Nolli ML., Corti A., Soffientini A., Cassani G., A monoclonal antibody that recognizes the receptor binding region of human urokinase plasminogen activator, Throm Haemostas 1986, 56:214-8), Mab105IF10 and Mab52C7 (see Salerno, G. et I., above) and rabbit anti-uPA antiserum.

### 1.6.5. - Treatment of pro-uPA with glycopeptidase F and neuraminidase

Aliquotes (2 microl) of recombinant scu-PA (pro-uPA) and urinary scu-PA, prepared substantially as described by Stump et al. see point 1.6.1, 1.5 mg/ml in 0.1 M sodium phosphate buffer pH 7.4 are mixed with 2 microl of a 5 microg/ml plasmin solution in the same buffer and left to stand for 4 h at 37°C. Then either 3 microl of 6.6 IU/ml glycopeptidase F (Boerhinger Mannheim) in 100 mM sodium phosphate, 20 mM EDTA (ethylen diamine tetracetic acid) 0.1% 2-mercaptoethanol, 1% Triton X-100 (polyethylene glycol p-isoctylphenyl ether) and 10 mM benzamidine, or 4 microl of 1 mg/ml neuraminidase (Clostridium perfringens, from Boehringer) in 50 mM sodium acetate buffer, pH 5, 50 mM sodium chloride and 10 mM benzamidine are added to each sample. All the samples are left to react overnight at 37°C and then analyzed by SDS-PAGE (see 1.6.4, above).

### 1.6.6 - Isoelectrofocusing (IEF)

IEF is performed in 1 mm thick gel slabs containing 5% acrylamide (the ratio acrylamide to bisacrylamide being preferably 25:1), 2% ampholine pH 3.5-10, 4M urea, and 3.5% glycerol. The gel is cooled at 12°C. 50 microl of sample (0.3 mg/ml) is applied on filter paper strips at the anode, after pre-focusing. The total running time is 150 min at 20 W, 1500 V at equilibration. pH measurements are made by cutting 5 mm slices of gel, eluting with 1 ml of 10 mM potassium chloride and measuring the pH at 12°C. No pI corrections are made for the presence of urea. The gel staining is carried out as described by Blakesley RW, Boezi JA, A new staining technique for proteins in polyacrylamide gels using Comassie Brilliant Blue G-250, Anal Biochem 1977; 82,580-582.

### 1.6.7 - N-terminal sequence analysis

N-terminal aminoacid sequence is performed using an applied Biosystem model 470A gas-phase protein sequencer.

### 1.6.8 - C-terminal sequence analysis

C-terminal analysis is performed by incubation of 3F11sc-uPA (RSV-LB6-scu-PA) (200 microgram) with carboxypeptidase Y Sigma, St. Louis, MO, USA) at room temperature in 100 microliter 0.1M sodium acetate pH 5.5 and 4M urea. The ratio of carboxypeptidase Y to 3F11sc-uPA is about 1:50 by mass. At intervals, 10 microliter aliquots are removed, heated at 100°C for 10 min and dried under vacuum. Each sample is dissolved in 40 microliter 0.2M sodium borate, pH 9.5, and portions of 5 microliter are treated with an excess of o-phthalaldehyde for 1 min at room temperature, and then loaded onto a Beckman Ultrasphere ODS (250 x 4.6 mm) column in a HP1090 HPLC system, equilibrated in 0.1M sodium phosphate, pH 6.8. Separation is achieved by a 23 min linear gradient from 40% to 80% aqueous methanol, at a flow rate of 0.7 ml/min. Aminoacid derivatives are identified with an HP1046 spectrofluorimeter and quantitated with an HP3390 integrator according to usual statistic analysis and calculation methods.

### 1.6.9 - Inhibition of tcu-PA activity by PAI-1 Human endothelial Plasminogen Activator Inhibitor

Human endothelial plasminogen activator inhibitor (PAI-1, 20 ug/ml) is activated with SDS (final concentration 0.5%) for 30 min at 37°C. To serial dilutions of activated PAI-1 (55 microl), 55 microl of 1% (v/v) Triton X-100 in 0.2 M Tris-HCl buffer, pH 7.6 and 0.1% (v/v) Tween 20 are added. Then, 30 microl of 8 ng/ml tcu-PA (test sample) are added, mixed and incubated for 30 min at room temperature. To measure the residual activity, 110 microl of human plasminogen (6 C.U./ml) in 0.1 M Tris-HCl, pH 7.6 and 0.1% Tween 20, are added and the samples are incubated at 37°C for 45 min. Then, 0.5 ml 0.9 mM S-2251 is added and the sample is incubated for 20min at room temperature. After stopping the reaction with 0.1 ml 50% acetic acid, the optical density at 405 nm is measured. The residual activity in the presence of different amounts of PAI-1 is expressed as a percentage of the activity of controls without PAI-1 and dose-response curves are plotted. From these plots the amount of PAI-1 at which 50% of the control plasminogen activator activity is retained is calculated.

### 1.7 - Kinetic analysis

### 1.7.1 - Activation of scu-PA's with Plasmin

The kinetics of the activation of urinary and recombinant scu-PA by plasmin is measured at 37°C in 50 mM Tris-HCl buffer, pH 7.6, containing 38 mM sodium chloride, 0.01 % Tween 20, 0.01 % bovine serum albumin and 0.5 mM pyro-Glu-Gly-Arg-p-nitroanilide (S-2444). The reaction system is calibrated for 0.5 nM plasmin and 50-750 nM scu-PA's. S-2444 is a specific substrate for tcu-PA and is not hydrolyzed by plasmin, hence under these conditions the tcu-PA generated by each scu-PA upon plasmin activation is proportional to the amount of p-nitroaniline released, detected photometrically at 405 nm. The initial activation rates, when plotted in a Lineweaver-Burk type of diagram, allow the calculation of the values of Km and Vmax.

### 1.7.2 - Amidolytic Activity on S-2444

To determine the kinetic parameters of the amidolytic activity, plasmin-activated u-PA's (4.5 nM) are incubated at different concentrations (0.03 - 0.3 mM) of pyro-Glu-Gly-Arg-p-nitroanilide (S-2444) in 0.1 M Tris Cl (pH 8.8), 50 mM NaCl and 0.1% bovine serum albumin at 37°C. The initial rate of p-nitroaniline release is determined photometrically at 405 nm. The linear increase of absorbance with time for the various concentrations of S-2444 is reported in a Lineweaver-Burk plot from which $K_m$ and $V_{max}$ are calculated by extrapolation of the curves obtained using a linear regression program.

### 1.7.3 - Activation of Human Plasminogen

The kinetics of the activation of plasminogen to plasmin by urinary and recombinant tc-uPA's (20 pM) is measured at 37°C in 0.05 M Tris-HCl buffer, pH 7.4, containing 0.04 M NaCl, 0.01% Tween 80, 0.01% bovine serum albumin, 0.5 mM D-Val-Leu-Lys-p-nitroanilide (2-2251) and plasminogen (5.2 - 23 mM). Since S-2251 is a specific substrate for plasmin and is not hydrolyzed by tc-uPA, in these conditions the generated plasmin activity is proportional to the amount of p-nitroaniline released, detected photometrically at 405 nm. The initial activation rates for the different concentrations of plasminogen are reported in a Lineweaver-Burk plot from which $K_m$ and $V_{max}$ are calculated by extrapolation of the curves obtained using a linear regression program.

Example E0

### Human Genomic Library

A library of human genomic DNA in bacteriophage lambda Charon 28 is prepared by techniques known in the art (c.f. P.H. Hieter et al., Cell, Vol. 22, 197-207 (1980) and Maniatis et al). The recombinant lambda lysate is titrated on E. Coli K12 strain LE 392 (Maniatis et al p. 504). Techniques for phage titration, as well as for screening of a phage library with a nick-translated probe, are also well known in the art (see for instance, Maniatis et al) and are applied substantially as such in the following experiments. For screening, 10 microliters of a one thousand fold dilution of the lysate are adsorbed onto 0.3 ml of a 2.5 fold concentrated culture of E. Coli K12 LE 392 in 10 mM $MgSO_4$ and incubated for 20 minutes at 37° C. 7 ml of 0.7% agarose is added and the whole mixture is layered onto 150 mm plastic plates containing NZYMC agar (type-A hydrolizate of caseine 10 g/l; NaCl 5 g/l; yeast extract 5 g/l; casamino acids 1 g/l; and $MgSO_4.7H_2O$ 2 g; adjusted to pH 7.5; see Maniatis et al). A total of 0.41 ml of a $10^{-3}$ dilution of the lysate is used, with a total of 41 plates. After one day incubation, an average of $1.5 \times 10^4$ plaques/plate is observed. The total of $6 \times 10^5$ plaques observed in the 41 plates is a good representation. All plaques from each plate are transferred to two nitrocellulose filters (Schleicher and Schuell Co.). The filters are dried, washed, baked in vacuum and pre-hybridized as usual (see Maniatis et al.). The hybridization is carried out with a nick-translated probe consisting of a DNA fragment isolated from a human urokinase cDNA (Verde A. et al., Proc. Natl. Acad. Sci., 81, 4727-4731, 1984)

### E0.1 - Nick Translation

Plasmid pHUK-I (Verde et al., Proc. Natl. Acad. Sci. 81, 4727-4731 1984) contains a recombinant insert complementary to the mRNA for human urokinase. A 1.5kb PstI fragment, isolated by agarose gel electrophoresis cutting out the corresponding gel region and recovering the DNA fragment by electro-elution (c.f. 1.5.7), contains about 75% of the coding region of the human urokinase mRNA (Verde et al., Proc. Natl. Acad. Sci., 81, 4727-4731, 1984). Uniform labeling of this PstI fragment (5.0 microg) is carried out with a nick-translation kit of the Bethesda Research Laboratories in the presence of 200 microCi each of alpha $^{32}$P-dCTP and alpha $^{32}$P-dGTP (c.f. Maniatis et al.). The yield of labelled DNA is $1.8-2.9 \times 10^8$ cpm/microg.

### E0.2 - Isolation of uPA gene positive phages

The 41 couples of filters (obtained as in EO) are hybridized with the nick-translated Pst-I fragment. Groups of 8 filters are hybridized in 40 ml of hybridization solution in a single plastic envelope with about $10^8$ cpm of labelled DNA. Hybridization is carried out at 42° C for 17 hours in 2 x SSC (17.5 g of NaCl and 88 g of sodium citrate are dissolved in 800 ml of water, the pH is adjusted to 7.4 with aquoues NaOH and the volume is adjusted to 1 l), 0.1% sodium dodecyl sulfate (SDS) (Maniatis et al, pag. 447). The filters are washed four times in 2 x SSC 0.1% SDS for 15 minutes at room temperature, and once in 1 x SSC, 0.1% SDS for 2 hours at 68° C. After drying, the filters are exposed to X-ray films (Kodak) for 12-24 h and developed. From this screening, the plaques that are found to give a positive hybridization spot in identical positions on both duplicate filters (three) are picked up with a small volume of NZYMC broth (Maniatis et al.) and used to infect E. Coli K 12 L 392 at different dilutions and spread on 10 cm plates of NZYMC-agar. The plates that give between 500 and 5000 plaques are then selected and re-screened. These plaques are transferred onto duplicate nitrocellulose filters (Schleicher and Schuell Co.) and the filters are treated and hybridized with the nick-translated 1.5 Kb Pst-I fragment of human uPA cDNA described above. Practically all plaques give a positive hybridization signal, with an enrichment factor of about $10^5$ .Single plaque isolates of the three positive plaques were named lambda-uPA-1 lambda-uPA-2 and lambda-uPA-3.

### E0.3 - Large scale phage preparation

The plaques are amplified on E. Coli K12 LE 392 using large culture lysates, the phages are isolated by repeated banding in cesium-chloride, purificated by extraction of the aqueous phase with phenol and then with chloroform/isoamyl alcohol (24:1) and DNA ethanol precipitation from the aqueous phase according to the procedure described by Maniatis et al., ch 3.

Example E1

Expression of uPA-coding gene in mammalian cells

### E1.1 - Isolation of the uPA ORF

200 microg of lambda-uPAI obtained essentially as in E0.3 are digested in 80 microl with 30 U of Smal at 25°C for 1 h; the reaction is stopped with EDTA (final concentration 50mM) and the DNA electrophoresed onto an ethidium bromide containing 0.8% agarose gel using a 10 cm comb at 100V. Then, the gel is analyzed under UV light and the second slowest band (of about 7.3 kb), a Smal fragment containing the open reading frame (ORF) of the human uPA gene, is cut out from the gel. The contained DNA is electroeluted and purified as described in 1.5.7.

### E1.2 - Preparation of vector DNA

A RSV-neo derived fragment containing the RSV promoter and the pBR 322 derived ampicillin resistance and origin of replication is prepared by HindIII digestion, filling in, Hpal digestion, CIP treatment and fragment separation on agarose gel.

More particularly, RSV-neo vector is modified as follows (see fig. 2):

### E1.2.1. - HindIII digestion

20 microg RSV-neo plasmid DNA is digested to completion with 80 U of HindIII for 1 h at 37°C in 200microl. The restriction enzyme is heat inactivated and then the mixture is extracted with an equal volume of phenol: chloroform, the DNA is precipitated adding also 50 microg tRNA (1.5.3), and re-dissolved in 20microl TE (Tris 10 mM pH.8 and EDTA 1 mM; Maniatis et al).

### E1.2.2 - Filling in and Hpal digestion

The protruding ends of the open vector prepared above are made flush by incubating the DNA with 1U of the Klenow fragment of DNA polymerase I in 25 microl (Maniatis et al, pag. 113) for 1 h at 37°C and purifying it as indicated in 1.5.2 and 1.5.3. Then the linearized, blunt-ended vector DNA is cleaved with 80 U Hpal in 40 microl for 1 h at 37°C and purified in the same manner. These operations cleave the neomycin resistance gene leaving a linear flush-ended 3.5 kb long DNA fragment having the RSV-Neo opened between the LTR promoter region and the SV40 polyadenilation site and containing the origin of replication and the ampicillin resistance gene derived from plasmid pBR322 (Fig. 2)

### E1.2.3 - CIP treatment

The DNA is treated with 1.5 U calf intestine phosphatase (CIP) in 50 microl reaction medium (final volume) in order to remove the 5' phosphate residue from the DNA ends; the enzyme is subsequently inactivated by adding ethylene bis(oxyethylenitrilo) tetracetic acid (EGTA) (final concentration 10 mM) and treatment at 68°C (see 1.5.4.)

### E1.2.4 - Fragment purification

DNA fragments are separated using a 0.6% agarose preparative gel as indicated in 1.5.7. The portion of the gel containing the 3.5 kb band is cut out and the DNA fragment is electroeluted (1.5.7), the obtained mixture is adjusted to the appropriate salt concentration and purified on a Elutip-D column (Schleicher and Schuell Co.). After elution, ethanol, Na-acetate, and carrier tRNA are added to the DNA solution and the DNA is precipitated and redissolved in 20 microl TE as described in 1.5.3.

### E.1.3-Cloning of the uPA gene under the RSV-LTR promoter

3 U of T4 DNA ligase and 2.5 microl of 10X buffer (Maniatis et al., p. 246) are added to 3 microg of dephosphorilated DNA vector (prepared as described above in E1.2) and 7.5 microg of 7.3 kb uPA fragment (prepared as described in E1.1). The 25 microl reaction mixture is incubated 16 h at 14° C (see 1.5.5) and used to transform 100 microl of HB101 E. coli competent cells (see 1.5.8). Bacteria are selected overnight on LB agar plates (tryptone 10 g/l, yeast extract 5 g/l, NaCl 10 g/l, adjusted to pH 7.5 with aqueous sodium hydroxide) containing 50 microg/ml ampicillin, at 37° C. Isolated colonies are picked up, grown in 5 ml ampicillin containing LB and processed by standard miniprep analysis (1.5.9). The restriction pattern of the purified plasmid DNAs are studied in order to establish the right orientation of the uPA gene in the vector frame. One of these plasmid having the correct orientation is designated RSV-uPA, isolated and used in further experiments (Fig. 2).

### E.1.4.1 - Transfection.

One day before transfections, the cells (murine LB6, described by Corsaro C.M. and Pearson L.M. in Somatic Cell Genetics, 7, 603, 1981), are plated at a concentration of about $10^4$ microl/cm$^2$ in Dulbecco's modified Eagle medium (DMEM, Flow Laboratories Inc.) supplemented with penicillin (50 U/ml), streptomycin (50 microg/ml), 2 mM glutamine, and 10% heat inactivated foetal calf serum (FCS). The day of transfection, the cells are in logarithmic growth phase and are refed 3 h before DNA addition. The calcium phosphate-DNA co-precipitation method is essentially followed for transfection (Graham and Van der Eb, Virology 52, 456, 1983). Calcium phosphate DNA precipitate is prepared by adding a DNA solution (in this case a mixture of RSV-uPA, see E1.3, and RSV-neo, see E1.2, about 1 to 1) in 2M CaCl$_2$ (0.25 ml, final volume) to a 2 X HBS (137mM NaCl, 0.7mM Na$_2$HPO$_4$, 21mM HEPES, 4-(2-hydroxyethyl)-1-piperazine ethansulfonic acid, pH 7.1) (0.25 ml). This DNA co-precipitate mixture is then added to the cells. After 3 h at 37° C, the precipitate is removed from the cells which are washed twice with DMEM (without foetal calf serum), 1.5 of a 15% solution of glycerol in HBS is added, the cells are incubated 1-2 min at 37° C and glycerol is removed by washing with DMEM (without foetal calf serum). Then the cells are fed with the complete DMEM reported above (when dealing with cell cultivation) and left at about 37° C for about 48 h, trypsinized very gently and plated at a concentration of about $10^5$ cell/plate in DMEM + 10% heat inactivated FCS + 50 U/ml penicillin, 50 microg/ml streptomycin, 2mM glutamin + 0.8 microg/ml G 418 (Geneticin, Gibco). Then, the cells are incubated for about 2-3 weeks, changing the medium every 3 days. After this time, colonies are counted, transferred onto 24-well microtiter plates, and allowed to grow up to saturation prior to assaying the supernatants. The clones which demonstrate useful levels of pro-uPA production (greater than 0.5 microg/ml) are stored as frozen stocks in liquid nitrogen after supplementing heat inactivated foetal calf serum with 10% DMSO. These cells are also re-cloned to select high producers which are also stored frozen as described above. The clone which gives the highest production of pro-uPA in these experiments (named 3F 11 or RSV-LB6) is recloned and mass cultivated.

### E1.4.2 - Mass cultivation.

A 4 liter cell reactor with automatic control of the dissolved oxygen tension, pH and temperature is used to grow an inoculum of the highest producing clone obtained according to the procedure described in the above examples, which was named 3 F 11, for convenience. The medium for the culture is DMEM with 5% heat-inactivated FCS, 1mM glutamine, 25U/ml penicillin, 25 microg/ml streptomycin, 400 microg/ml G 418 and 27 IU/ml aprotinin or a serum-free formulation containing DMEM and F10 (Flow Labs. Inc.), ratio 1:1, as the base supplemented with 27 IU/ml aprotinin, albumin, insulin, transferin, ethanolamine, choline, vitamins and trace metals.The cell inoculum is grown in a 150 cm$^2$ flasks in DMEM plus 10% heat-inactivated FCS, 2mM glutamine, 50 U/ml penicillin, 50 microg/ml streptomycin, 400 microg/ml G 418, and the cells are cultivated on Cytodex microcarriers (Pharmacia).

The cells attained a maximum population density of 2.5.10$^6$/ml and pro-uPA is synthesized throughout the duration of the culture and assayed by known methods such as the fibrinolytic or the IAA assay (see 1.6.2). A maximum concentration of 20 microg/ml was reached as tested by IAA.

### E1.4.3

The cloning experiments reported above (c.f. E1.4.1. and E1.4.2) are repeated using CHO (Kao F.T., Puck F., Proc. Natl. Acad. Sci. USA, 60, 1275-1281, 1968) or A 431 (Fabricant R.N. et al, Proc. Natl. Acad. Sci. USA, 74, 565-569; 1977) cells. The transfection/cultivation conditions are substantially unaltered apart from the substitution of Ham's F10 (Flow Labs) as the culture medium instead of DMEM for CHO cells.

The single chain uPA produced by CHO cells transfected with a RSV vector as described above is conveniently named RSV-CHO-scu-PA while that produced by A431 is named RSV-A431-scu-PA.

Recloning of prouPA producing mono-layer CHO clones and selection of non-adhesive producing clones gave a clone capable of producing pro-uPA in suspension (1.5 mg/l, by IAA).

### E1.5 - Quantitation of pro-uPA

The amount of pro-uPA produced by the cell lines obtained as described above (E 1.4) is either assayed via fibrinolysis (see 1.6.2) or by the immunoamidolytic assay (see 1.6.2). The results of transfection experiments made co-transfecting the plasmid containing the gene for the resistance to neomycin (RSV-neo) under the control of the Long Terminal Repeat of the Rous Sarcoma Virus (RSV-LTR) and the plasmid containing the uPA gene under the control of the same RSV-LTR (plasmid RSV-uPA) are reported in the following Table:

TABLE 1

| Cell line | No. of G 418 resistant clones | No. of Pro-uPA clones | No. of selected clones | pro-uPA Production (microg/ml) (by IAA) |
|---|---|---|---|---|
| LB6 | 28 | 1 | 1 | 0.2-2 |
| LB6 | 50 | 3 | 1 | 0.8-2 |
| LB6 | 158 | 14 | 3 | 1-5 <br> 1.5-5 <br> 2-10 |
| The clone producing 2-10 microg/ml of pro-uPA carrying from EXP 3 was subsequently subcloned and grown in mass culture for the production of pro-uPA. | | | | |

TABLE 1 (continued)

| Cell line | No. of G 418 resistant clones | No. of Pro-uPA clones | No. of selected clones | pro-uPA Production (microgram/ml) (by IAA) |
|---|---|---|---|---|
| CHO | 222 | 118 | 5 | 0.5-1<br>0.5-1.5<br>1-5<br>1.5-5<br>2-10 |
| A 431* | 2 | 2 | 1 | 5-10 |

* The "non-transformed" cells have a pro-uPA production level of 0.5 microgram/ml

Example E2

## Production of uPA by RSV-uPA-BPV vectors

In the RSVuPA plasmid DNA the restriction endonucleases NdeI and ApaI cut only once each (Fig.3), the first in the pBR322 derived region close to the RSV promoter and the second in the region 3' to the uPA polyadenilation site. These two sites can be modified by XhoI linkering to obtain, after XhoI digestion, a fragment containing the RSV promoter/uPA coding region suitable for cloning it in a vector having a XhoI site or a SalI site.

E2.1.1 - 70 microg of RSV-uPA DNA are digested to completion with 420 U NdeI in 300 microl for 24 h at 37°C (assay conditions: 150mM NaCl, 10mM Tris.HCl pH 7.8,7mM $MgCl_2$, 6mM 2-mercaptoethanol, 100 microg/ml BSA). After heat-inactivation, the NaCl concentration is adjusted to ApaI conditions (6mM NaCl, 6mM Tris.HCl pH 7.4, 6mM $MgCl_2$, 6mM 2-mercaptoethanol, 100 microg/ml BSA) and the DNA is completely cleaved with 160 U of ApaI in 400 microl (final volume) in 4 h at 30°C. The DNA is then purified and precipitated as in 1.5.3, redissolved in 20 microl of TE and the NdeI protruding ends are filled in with 5 U E. Coli DNA Polimerase I incubating the DNA (final reaction volume 25 microl) for 1 h at 37°C followed by heat inactivation of the enzyme (Maniatis et al, page 113).

E2.1.2 - The NdeI/ApaI digested, blunt-ended RSV-uPA plasmid as obtained above is mixed to phosphorilated XhoI linker DNA (molar ratio about 1:50) and incubated 18 h at 8°C, (final volume 50 microl), as indicated in 1.5.5, followed by ligase heat-inactivation. Then the DNA is digested with 120 U of XhoI in 200 microl for 4 h at 37°C and XhoI is heat-inactivated.

E2.1.3 - The DNA fragments obtained above are separated by electrophoresis on an ethidium bromide containing, 0.75% agarose gel and run 20 h at 35 V (see 1.5.7 for further details), the otained bands are detected on an UV transilluminator, the largest corresponding to the 8.35 kb RSV promoter/uPA coding region fragment and the smallest to the 2.45 kb ampicillin resistance and origin of replication containing-fragment. The two bands are cut out and purified as in 1.5.7. The smallest band is treated with 3 U of CIP for 30 min at 37°C and 30 min at 56°C and purified and precipitated as described in 1.5.2 and 1.5.3. The large and the small fragments are mixed in different molar ratios ranging from about 1:1 to about 4:1, ligated 3 h at 14°C and a fraction of each reaction mixture is used to transform E. Coli DH1 competent cells as described in 1.5.8. Bacteria are grown overnight on ampicillin containing agar plates, plasmids from isolated colonies are analyzed for the appropriate restriction fragment pattern and one of them is selected (plasmid p71) in which the human uPA gene placed under the RSV promoter translational control is excisable from the rest of the plasmid by cleavage with XhoI (Fig. 3 bottom).

E2.1.4 - BPV-restriction sites modification

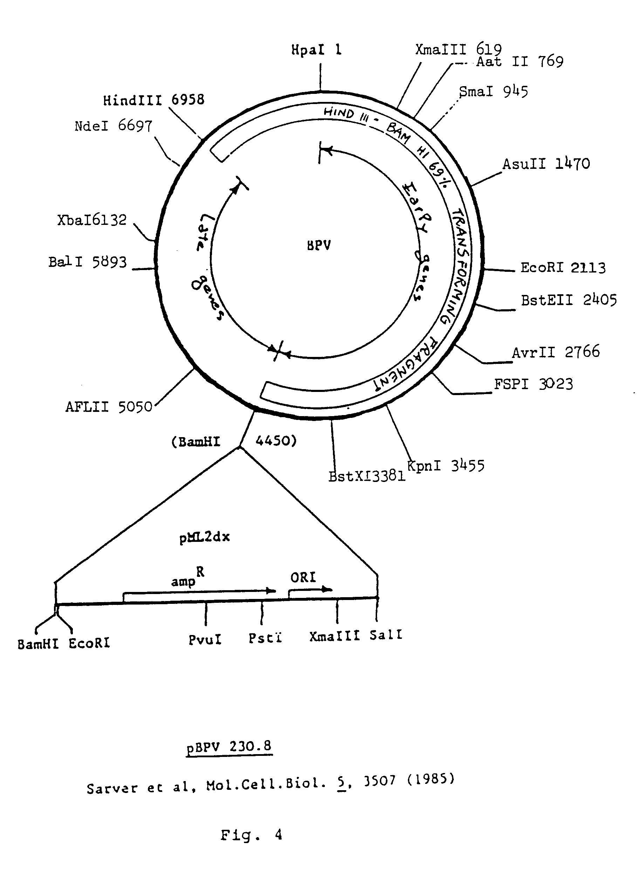

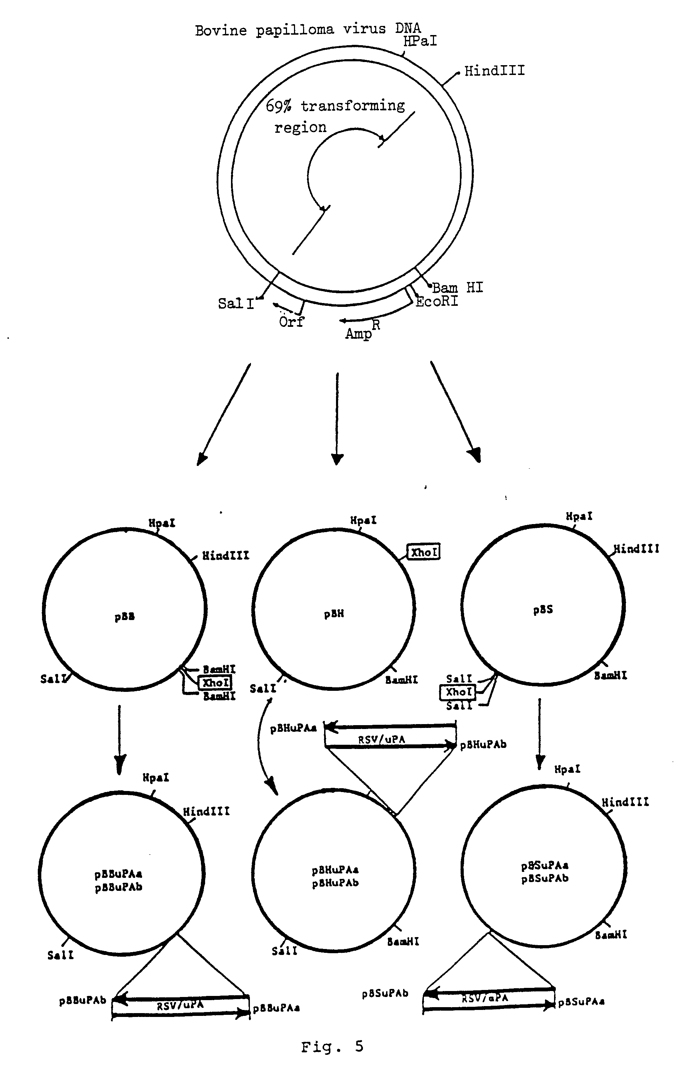

pBPV 230.8 (Fig. 4) is modified at three different sites (BamHI, Hind III or SalI, respectively) by inserting a unique XhoI site that make the insertion of the RSV-promoter/uPAcoding region (RSVuPA) easier. In each experiment, an excess of the appropriate restriction enzyme (BamHI, or HindIII or SalI) is used to digest to completion 10 microg of BPV230.8 DNA for 3 h at 37°C in 100 microl (final volume). The enzyme is heat-inactivated and the DNA is purified as described in 1.5.3, re-dissolved in 10 microl of buffer (50mM Tris pH 7.2 10mM MgSO₄, 0.1mM dithiothreitol, 50 microg/ml BSA; Maniatis et al, page 113) and rendered flush-ended by treating with 5 U of of the Klenow fragment of E. Coli DNA polimerase I for 2 h at 37°C. After polymerase heat inactivation, the DNA is dephosphorilated in 50 microl (final volume) with 5 U of CIP for 30 min at 37°C and for further 30 min at 56°C. Finally, this enzyme is inactivated by heating in the presence of EGTA and the DNA is purified as described in 1.5.2 and 1.5.3. To the redissolved blunt-ended, linear BPV230.8 DNA, labelled XhoI linker DNA is added (molar ratio 200:1) and ligated in 50 microl (final volume) for 15 h at 8°C in the presence of 2.5 U of T4 DNA ligase (1.5.4). After ligase heat inactivation, a sample containing about 1% of the ligated DNA is digested with 8 U of XhoI, electrophoresed with an equal amount of DNA aliquoted before and after the ligation and analyzed as in 1.5.6. Then, salt concentration is adjusted to 100mM NaCl and the pH is adjusted to about 7.5 (Maniatis et al, pag. 104 and Biochemicals for Molecular Biology; Boehringer Mannheim, Munich 1985), all the DNA is cleaved with XhoI, 1 h at 37°C in 200 microl, loaded on 0.6% agarose gel, electrophoreted and the band corresponding to the linearized plasmid cut out, electroeluted and purified as in 1.5.7. The linear DNA is self-annealed by ligation and used to transform competent E. Coli DH5 cells (BRL) (see 1.5.8). Plasmids from colonies isolated after overnight incubation on ampicillin containing agar plates are screened by miniprep analysis (1.5.9) for the presence of the new XhoI site. The following pBPV230.8 derived plasmids were obtained (Fig. 5, in the middle):
- pBB, wherein a XhoI site is created at the BamHI position and is flanked by two BamHI sites;
- pBS, wherein a XhoI site is created at the SalI position and is flanked by two SalI sites;
- pBH, wherein a XhoI site is created at the HindIII position.

E2.1.5 - In different experiments, 50 microg each of pBB, pBH and pBS DNAs are digested to completion with an excess of XhoI (SalI in the case of pBS) in 50 microl, at 37°C for 2 h, the enzyme is heat-inactivated, and the linearized plasmid DNA is dephosphorilated with 1U CIP as described in 1.5.4. After enzyme heat-inactivation, the DNA is loaded on a ethidium bromide containing, 0.6% agarose gel and electrophoreted for 16 h at 30V. A major DNA band corresponding to the monomeric linear plasmid is electroeluted, purified and re-dissolved as described in 1.5.7. Similarly, 50 microg of p71 plasmid DNA is digested to completion with 80U of XhoI, the enzyme is heat-inactivated, and the mixture is loaded on an ethidium bromide containing 0.6% agarose gel and electrophoresed for 16 h at 30 V. The gel portion with the 8.35kb band, which contains the RSV promoter/uPA coding region construct, is electroeluted, purified and redissolved as described in 1.5.7. BPV vector DNA (Fig. 5, in the middle) and insert DNA (Fig. 3, bottom) are mixed in ratios varying from 2:1 to 1:4 and ligated with 2U/sample of T4 DNA ligase for 24 h at 15°C at a DNA concentration of approximatively 0,2 microg/microl. The reaction medium is adjusted with the buffer and the ligated DNA is digested 1 h at 37°C with 20 U of XbaI in 15 microl (final volume). (Assay conditions: 50mM NaCl, 6mM Tris-HCl pH 7.9, 6mM MgCl₂, 100 microg/ml BSA). This enzyme does not cleave the RSV/uPA construct and cut the BPV230.8 derived vectors only once. After XbaI heat-inactivation the obtained DNA mixture is diluted 1 to 5 with water, 10x ligation buffer is added, and incubated 2 h at 14°C with 2.5 U of T4 DNA ligase. A 5 microl sample from each reaction experiment is used to transform E. Coli DH5 competent cells (1.5.8). These bacteria are grown overnight on ampicillin-containing agar plates at 37°C and samples from the resulting colonies are analyzed by miniprep analysis (see 1.5.9). Plasmids from isolated colonies with the proper restriction pattern are selected (1.5.9, Fig. 5 bottom).

E.2.1.6. - Transfection/mass cultivation

Transfection experiments in murine LB6 cells are performed as described under E.1.4.1 and the highest producing cells are mass cultivated as indicated in E.1.4.2.

25

E.2.1.7. - Quantitation of uPA

The amount of uPA produced by the cells transfected according to E.2.1.6 is evaluated as indicated under E.1.5. Transfections made co-transfecting the RSV-Neo plasmid containing the gene for the resistance to neomycin (neo) under the control of the long terminal repeat of the Rous Sarcoma Virus (RSV-LTR) and the BPV derived vectors containing the RSV-uPA cassette (Fig. 3 bottom) (see E 2.1.5) gave neo-resistant, pro-uPA-producing clones with pro-uPA levels ranging between 1.3 microg/ml and 26 microg/ml. The single chain uPA produced by these cells is named BPV-LB6-scu-PA. The data relating to these experiments are reported below:

| No. of G 418 resistant clones (microgram/ml) | No. of Pro-uPA clones | No. of selected clones | pro-uPA Production (by IAA) |
|---|---|---|---|
| 1 | 96 | 5 | 1-3 |
| | | | 1-5 |
| | | | 2-10 |
| | | | 2-10 |
| | | | 10-20 |

Example E3

Purification and characterization of pro-uPA

The supernatants of cultivated transformed cells (see E1.4.2) are separately purified and then analyzed to ascertain their main structural features. The electrophoretic behaviour of RSV-LB6-scu-PA, RSV-A431-scu-PA, RSV-CHO-scu-PA, and BPV-LB6-scu-PA is compared in parallel with scu-PA of human urinary origin and scu-PA of A431 untransformed cells (A431-scu-PA).

E3.1 - Purification of pro-uPA

3F11-scu-PA (RSV-LB6-scu-PA), RSV-A431-sc-uPA, RSV-CHO-sc-uPA and BPV-LB6-sc-uPA (see E1.4.2) are separately purified from cell culture supernatants by immunoaffinity chromatography on Mab5B4-agarose and ion exchange FPLC by essentially following the procedure as previously described for the purification of A431-uPA from A431 tumor cells supernatants (Corti A., Nolli M.L., Soffientini A., Cassani G, Purification and characterization of single-chain urokinase-type plasminogen activator from human A431 cells, Throm Haemostas 1986; 56:219-224).

An agarose modified matrix bearing a monoclonal antibody which binds the high molecular weigh urokinase without binding the low molecular form (MAB 5B4-agarose, 2.6 cm x 8.6 cm. prepared according to conventional methods as described by Corti A., Nolli ML., Soffientini A. and Cassani G. in Throm Haemostas 1986; 56:219-24), is equilibrated with 0.15 M sodium chloride, and 0.05 M sodium phosphate buffer pH 7.4 containing 1% polyethylenglycol (PEG) 6000. The column is loaded with 24 liters of a scu-PA containing cell supernatant (see above and E1.4.2) at a flow rate of 220 ml/h, at 4°C, and then washed with the equilibrating buffer. Desorption is carried out with 1 M sodium chloride and 0.1 M acetic acid containing 1% PEG 6000. All fractions are tested by IAA and those fractions which contain a scu-PA are collected, pooled and further purified and concentrated by ion exchange chromatography in an FPLC (fast protein liquid chromatography) system (Pharmacia Fine Chemicals, Sweden) using a Mono S HR5/5 column (strong cation exchanger based on beaded hydrophilic resin with narrow particle size distribution; the charged group is -$CH_2SO_3$) pre-equilibrated with 0.05 M sodium acetate buffer, pH 4. The sample is generally two-fold diluted with distilled water to reduce the ionic strength and then loaded on the column. After washing with the equilibrating buffer, the bound proteins are eluted with a salt/pH linear gradient mixture of buffer A (0.05 M sodium acetate buffer, pH 4) and buffer B (1 M sodium chloride, 0.05 sodium acetate, pH 5) from 30 to 70% B in A in 50 min, then 100% B for 20 min; flow rate, 60 ml/h. 3F11 (RSV-LB6) cell supernatants contained 0.034 mg/l of enzymatically active uPA antigen and 0.870 mg/l of enzymatically inactive, plasmin activable uPA antigen as measured by immunoadsorbent-amidolytic assay (IAA) (see 1.6.2). The amounts of enzymatically active and inactive uPA (pro-uPA) recovered at each step are reported in Table 2. About 0.95 mg of active uPA and 15.5 mg of plasma activable uPA was recovered from 24.1 l of 3F11 supernatant corresponding to overall yields of 116% and 73.8% respectively, as evaluated by IAA. The enzymatically active uPA in the final product amounted to 5.8% of the total. Reverse-phase FPLC analysis of 3F11-scu-PA (RSV-LB6-scu-PA) resulted in a single peak of adsorbance at 280 nm with the same retention time as A431scu-PA and urinary scu-PA (c.f. 1.6.1).

Similar results are obtained when cell supernatants (c.f. E1.4.2) from PSV-A431-scu-PA, RSV-CHO-scu-PA and BPV-LB6-scu-PA producing cells are submitted to this purification procedure.

E3.2 - Structural characterization of scu-PA

E3.2.1 - Molecular weight and immunoreactivity

The material purified from 3F11 cell supernatants (3F11 scu-PA) (see E1.6) is analyzed by SDS-PAGE and immunoblotting in order to evaluate its purity, molecular form and immunological properties. SDS-PAGE (see 1.6.4) of 3F11-uPA under non-reducing conditions revealed a 52,000 Mr band that after blotting on nitrocellulose membrane immunoreacted with rabbit anti-uPA antiserum and various monoclonal antibodies against different epitopes of urinary uPA, such as Mab5B4, Mab 105IF10 and Mab 52C7 (see 1.6.4). A similar immunoreactivity pattern was obtained also with natural urinary scu-PA (1.6.1) indicating that 3F11 scu-PA is immunologically closely related to human urinary scu-PA. SDS-PAGE of 3F11-uPA under reducing conditions revealed a main band at 50,000 Mr corresponding to more than 90% of the total protein, and two minor bands at 33,000 Mr and 20,000 Mr. All bands immunoreacted with anti-uPA antiserum by Western blot indicating that the material purified from 3F11 cell supernatants consists essentially of a single-chain uPA (3F11 scu-PA) 50,000 Mr band with less than 10% of two-chain uPA (3F11tcu-PA), 33,000 Mr and 20,000 Mr bands.

E3.2.2 - Chain structure and glycosylation

The molecular form of 3F11-scu-PA (RSV-LB6-uPA), RSV-A431-scu-PA, RSV-CHO-scu-PA and BPV-LB6-scu-PA is investigated after activation with plasmin (c.f. 1.6.2). All the above substances (scu-PA's) are totally converted by plasmin into 52000 Mr two-chain proteins (tcu-PA's) made up by a 20,000 Mr A-chain and a 33,000 Mr B-chain as evaluated by SDS-PAGE under reducing conditions. The molecular weight and chain structure of recombinant scu-PA's were compared with those of natural urinary scu-PA (1.6.1) before and after treatment with plasmin and glycopeptidase F or with plasmin and neuraminidase (see 1.6.5).

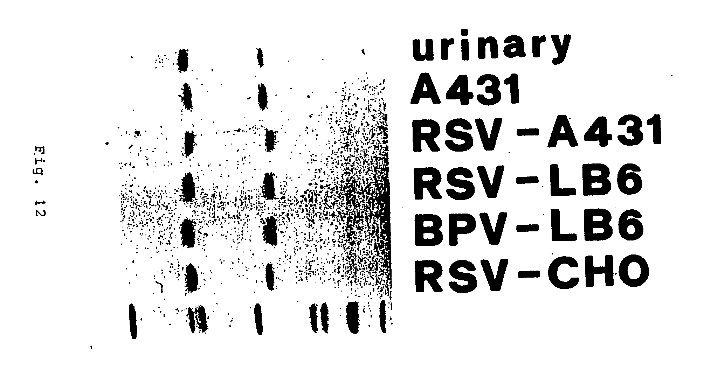

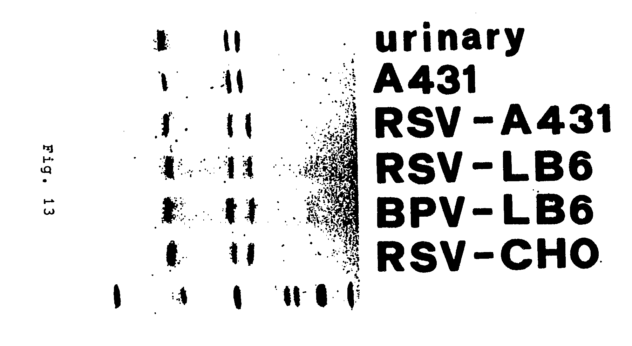

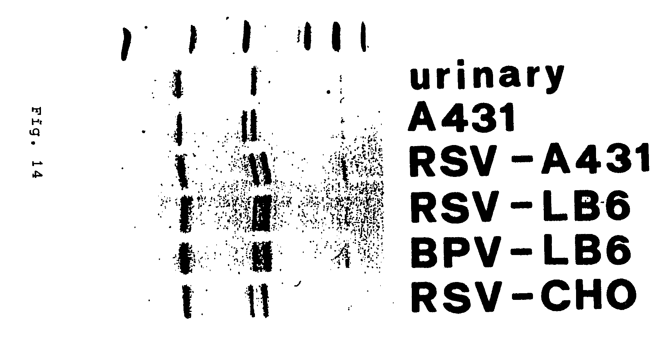

The electrophoretic mobility of each of the above reported recombinant scu-PA's under non-reducing conditions was slightly lower than that of urinary scu-PA corresponding to a difference of 1000-2000 in molecular weight (see Figure 11). A similar result was also obtained under reducing conditions. The molecular weight of the B-chain of tcu-PA's, as calculated on the basis of its mobility, resulted 32,000-33,000 Mr while that of the B-chain of urinary tcu-PA resulted 31,000 Mr (see Figure 12). No differences were observed on A-chains (20,000 Mr). When all the scu-PA's were treated with glycopeptidase F to hydrolyse the N-glycosidic bonds (Steffens G.J., Günzler W.A., Otting F., Frankus E., Flohe L. The complete aminoacid sequence of low molecular mass urokinase human urine, Hoppe-Seyler's Z. Physiol Chem. 1982, 363, 1043-1058) identical bands of 30000 Mr were observed for the B-chains of all proteins (see Figure 13). These data suggest that the difference in electrophoretic mobility of recombinant scu-PA's and urinary scu-PA is related to a different sugar content. This difference in sugar content was confirmed by treatment of the above reported scu-PA's with neuraminidase (see 1.6.5.), an enzyme that selectively cuts off the terminal sialic acid residues of glycoproteins. After incubation with plasmin and then with neuraminidase, urinary scu-PA showed no detectable difference in both the A and B chains when compared with urinary scu-PA treated with plasmin only. Instead, the B-chain of recombinant scu-PA's, in the same conditions, showed an electrophoretic mobility intermediate between that of the untreated protein and that of the fully deglycosylated protein (see Figure 14). Therefore these proteins have different sialic acid content.

### E3.2.3 - Isoelectrofocusing

3F11scu-PA and RSV-A431-scu-PA were subjected to IEF (see 1.6.6) in comparison with A431scu-PA and urinary scu-PA. Several isoelectric forms were observed for all proteins; in particular at least six isoelectric forms of pI 8.75, 8.85, 9.0, 9.1, 9.15, and 9.25 were observed 3F11scu-PA and RSV-A431-scu-PA while only four isoelectric forms of pI 9.1, 9.15, 9.25 and 9.3 were observed for A431scu-PA. Urinary scu-PA presented all the bands observed with 3F11scu-PA and A431scu-PA, though in different amounts.

### E3.2.4 - Aminoacid sequence analysis

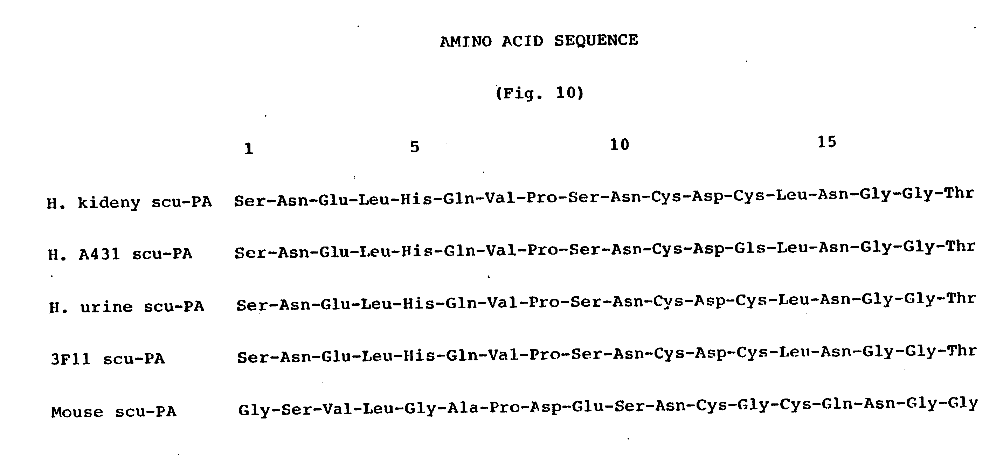

A) The N-terminal aminoacid sequence (18 residues) of 3F11scu-PA was analyzed by automated Edman degradation (see 1.6.7). The sequence obtained is reported in Fig. 10. As shown, the sequence of 3F11scu-PA is identical with that of natural human urinary scu-PA and A431scu-PA (Kasai S., Arimura H., Nishida M., Suyama T. Primary structure of single-chain pro-urokinase, J. Biol. Chem. 1985; 260/12382-12389), and clearly distinct from that of mouse scu-PA (Belin D., Vassalli J.D., Combepine C., Godeau F., Nagamine Y., Reich E., Kocher H.P., Duvoisin R., Cloning, nucleotide sequencing and expression of cDNAs encoding mouse urokinase-type plasminogen activator, Eur J. Biochem. 1985, 148,225-232).

B) A kinetic analysis of the digestion of 3F11sc-uPA with carboxypeptidase Y (see 1.6.8) showed the C-terminal sequence Ala-Leu(COOH), in agreement with that of human urinary sc-uPA (Kasai S., Arimura H, Nishida M, and Suyama T Primary structure of single-chain pro-urokinase, J. Biol. Chem. 1985, 260, 12382-12389).

### E3.2.5 - Interaction of u-PA with PAI-1

Human endothelial Plasminogen Activator Inhibitor (PAI-1) reacts with tcu-PA to form a 1:1 molar complex which in a second step becomes covalent, thereby preventing the proteolytic activity of tcu-PA (Bachmann F, Fibrinolysis, In: Thrombosis and Haemostasis 1987, edited by M. Verstraete, J. Vermylen, H.R. Lijnen and J. Arnout, International Society on Thrombosis and Haemostasis and Leuven University Press, Leuven 1987, pp 227-265). We have investigated the extent of inhibition of the above reported uPA's (RSV-LB6-scu-PA, RSV-A431-scuPA, RSV-CHO-scuPA and BPV-LB6-scuPA after plasmin activation) by PAI-1 (see 1.6.9). For that purpose, increasing amounts of PAI-1 are preincubated with a fixed amount of plasmin-activated tcu-PA (see 1.6.2) and the residual plasminogen activator activity is measured with an indirect assay, using the plasmin-specific substrate S-2251. Dose-response curves of inhibition (percentage of the activity without PAI-1 as a function of PAI-1 concentration) are plotted. With 1.7 ng/ml, uPA 50% inhibition of activity was achieved in all cases with 6-8 ng/ml PAI-1, all tcu-PA's showing similar curves.

### E3.3 - Enzymatic Activity

### E3.3.1 - Fibrinolytic activity

The enzymatic activity of 3F11scu-PA was evaluated after conversion into enzymatically active 3F11tcu-PA by treatment with plasmin agarose (see 1.6.2). The immobilized plasmin can be easily separated from the activated enzyme by centrifugation, thus avoiding possible interferences in subsequent assays. The specific fibrinolytic activity of 3F11tcu-PA, as evaluated by the fibrin plate method, resulted 117225 IU/mg (see Table 2). This result is in good agreement with the reported values for A431tcu-PA (Corti A, Nolli ML, Cassani G, Differential detection of single-chain and two-chain urokinase type plasminogen activator by a new immunoadsorbent amidolytic assay (IAA), Throm Haemostas 1986; 56, 407-410).

## E3.3.2 - Kinetic Analysis

3F11-scu-PA (RSV-LB6-scuPA), RSV-A431-scuPA, RSV-CHO-scu-PA and BPV-LB6-scu-PA were compared with urinary scu-PA and A431-scu-PA for their kinetic behaviour in purified systems (see 1.7.1, 1.7.2 and 1.7.3). The activation of scu-PA's to tcu-PA's by plasmin (Table 3), and the ability of the plasmin-activated uPA's to hydrolyze the chromogenic substrate S-2444 (Table 4) and to activate plasminogen (Table 5) were studied with a Lineweaver-Burk type of analysis. In all cases the various scu-PA's showed a similar kinetic behaviour with comparable values of the Michaelis-Menten constant ($K_m$) and the catalytic constant ($k_{cat}$).

### TABLE 3

| Kinetics of Activation of scu-PA's with Plasmin (pH 7.6 and 37°C ) | | |
|---|---|---|
| scu-PA's | $K_m$ (microM ) | $k_{cat}$ ( $s^{-1}$ ) |
| urinary | 0.17 | 0.09 |
| A431 | 0.21 | 0.09 |
| RSV - A431 | 0.28 | 0.11 |
| RSV - LB6 | 0.14 | 0.11 |
| BPV - LB6 | | |
| RSV - CHO | | |

### TABLE 4

| Kinetics of Amidolytic Activity on Glp-Gly-Arg-pNA (S-2444) (pH 8.8 and 37°C ) | | |
|---|---|---|
| scu-PA's | $K_m$ (microM ) | $k_{cat}$ ( $s^{-1}$ ) |
| urinary | 80 | 25 |
| A431 | 72 | 23 |
| RSV - A431 | 76 | 23 |
| RSV - LB6 | 69 | 24 |
| BPV - LB6 | 78 | 26 |
| RSV - CHO | 76 | 26 |

### TABLE 5

| Kinetics of Activation of Human Plasminogen (pH 7.4 and 37°C ) | | |
|---|---|---|
| scu-PA's | $K_m$ (microM ) | $k_{cat}$ ( $s^{-1}$ ) |
| urinary | 23 | 1.1 |
| A431 | 17 | 1.0 |
| RSV - A431 | 21 | 1.1 |
| RSV - LB6 | 24 | 1.3 |
| BPV - LB6 | 24 | 1.3 |
| RSV - CHO | 24 | 1.3 |

Example E4

## Construction of modified derivatives of the human uPA promoter region

### E4.1 - Construction of the puPA2 plasmid

50 microg of lambda-uPA1 DNA prepared as described in E0.3 are digested in 100 microl (final volume) with 30 U of SmaI in the appropriate buffer for 60 min at 30° C. 500 ng of the obtained DNA are analyzed on an ethidium bromide containing 1% agarose gel while the remaining is stored at -20° C. The enzyme is then heat-inactivated at 68° C (10 min) and the resulting DNA preparation is loaded on an ethidium bromide containing 0.8% preparative agarose gel. The electrophoretic separation is runned 20 h at 50 V in TBE buffer (Maniatis et al, ch. 5) and the DNA bands are visualized on a medium-lenght UV transilluminator. A band of approximatively 2.38 kb that contains the pro-uPA promoter is cut out and the DNA electroeluted and purified as described in 1.5.7. 50 microg of pEMBL8CAT DNA (Fig. 6) is similarly digested for 1 hour at 30° C with 30 U of SmaI in 25 microl (final volume). 64 Microl of water, 10 microl of CIP 10X buffer and 1 U of CIP in 1 microl (final volume) are added and the reaction mixture is further incubated for 60 min at 37° C. CIP is inactivated adding EGTA and heating as described in 1.5.4 and the DNA is purified on an ethidium bromide containing, 0.8% agarose gel under the same conditions described in 1.5.7. 1.5 microg of the 2.38 kb lambda-uPA1 derived fragment and 500 ng of linearized, dephosphorylated pEMBL8CAT DNA as prepared above are mixed togheter and ligated in 20 microl of the appropriate buffer containing 10% PEG and 2 U of T4 DNA ligase for 20 hours at 14° C. 5 microl of this reaction mixture are then used to transform competent HB101 E.Coli cells as described in 1.5.8. Ampicillin resistant colonies are analyzed, and among those containing the insert in the correct orientation one is selected and designated puPA2. (Fig. 6 on the right).

### E4.2 - Construction of the puPA2/41 plasmid

Optimal conditions to obtain a SmaI partially digested, linearized form of puPA2 DNA prepared above are investigated by digesting 250 ng of this plasmid respectively with 1.0, 2.0, 3.0 and 4.0 U of SmaI in 10 microl (final volume) of the appropriate buffer. 50% (v/v) each reaction mixture is transferred to a different tube and frozen in a dry ice/ethanol bath after 6 min at 30° C of reaction, while the remaining portions are frozen after incubating 9 min longer. Then, each frozen sample is brought to 10 mM with EDTA, the enzyme is heat-inactivated and the DNA is analyzed on an ethidium bromide containig, 1% agarose minigel. Good results are achieved by incubating with 2 U for 15 min and with 3 U for 6 min. Then a preparative digestion is set up as follow: 5 microg of puPA2 DNA is digested with 40 U of SmaI in 50 microl of the appropriate buffer, the reaction is stopped by adding 1 microl of 500 mM EDTA and freezing. After heat-inactivation of the added enzyme this puPA2 DNA preparation is mixed with 2.25 microg of XhoI linker, phosphorylated as described in 1.5.6 and ligated in 86 microl of a reaction mixture containing 10% PEG 1500 and 2.5 U of T4 DNA ligase, incubating first 6 h at 7° C then increasing slowly the temperature to 17° C and incubating for further 10 hours. The ligating enzyme is heat-inactivated, 10% of the ligated DNA is digested with 55U of XhoI for 2 h at 37° C PRTin 20 microl (final volume), this enzyme is then heat-inactivated and the DNA precipitated as indicated in 1.5.3. The precipitated DNA is re-dissolved in 4 microl TE (Maniatis et al), 1 microl of 1U T4 DNA ligase containing 5X ligation buffer (Maniatis et al,) is added, the mixture is left to self-ligate at 15° C for 2h30 min and then used to transform HB101 competent E.Coli cells (1.5.8). Ampicillin resistant colonies are screened by miniprep analysis. One of these, having the SmaI site (which flanks the 5' end of the uPA promoter containing the 2.38kb insert) converted into an XhoI site (Fig. 8) is named puPA2/41.

### E4.3 - CAT analysis

puPA2, its derivative puPA2/41 and RSV-CAT are tested for their ability to drive the trascription of the CAT gene. Plasmid DNAs are transfected into human A1251 cell-line (1.1) which was derived from a kidney carcinoma (Stoppelli M.P., Verde P., Grimaldi G., Locatelli E.K., Blasi F., J. Cell Biol. 1986, 102, 1231-1241) by calcium phosphate precipitation procedure and the levels of the CAT protein possibly produced in the transfected cells are measured after 64 ± 4 hours.

### E4.3.1 - Purification of plasmid DNA

Plasmid DNAs are purified using the alkaline lysis method as described by Birnboim H.C. and Doly J. 1979, Nucleic Acids Res. 7, 1513 (reported also in Maniatis et al. p. 90) from a 400ml LB broth overnight culture of each bacterial clone. The plasmid DNA thus obtained is purified twice on ethidium bromide CsCl gradients, the band containing it is collected by punturing, and extensively extracted with isoamylic alcohol (see Maniatis et al. p.93-94). The DNA containing CsCl aqueous solution is then diluted 1:4 with water and the DNA is precipitated by adding 0.1 vol of 3M sodium acetate pH5.2 and 3 vol of ethanol. After leaving the mixture 30 min at -20°C, the DNA fraction is spinned down at 10000Xg for 15 min at 4°C, washed once with 70% ethanol and once with 100% ethanol, dried, dissolved in 2ml of TE, re-precipitated and washed in the same manner and finally re-dissolved in 1ml TE (Maniatis et al). O.D.$_{260}$ is measured and the concentration is adjusted to about 500ng/microl, checked on minigel and measured by spectrophotometry again. Finally the plasmid DNAs are diluted 1:1 with absolute ethanol and stored at -20°C.

### E.4.3.2 - Precipitation by calcium phosphate procedure

For each 60mm Petri dish to be transfected, 40 microl of DNA ethanol/TE solution, equivalent to 10 microg DNA, are dried in a Savant vacuum centrifuge and re-dissolved in 220 microl of water. 30 microl of 2M CaCl$_2$ (from Mallinkrodt or Merck) are added and quickly mixed. 250 microl of 2X HBS (10g/l HEPES, 4-(2-hydroxyethyl)-1-piperazine ethanesulfonic acid, 16g/l NaCl, pH 7.1 ±0.05, sterile) and 5 microl of 100X PO$_4$ (70mM Na$_2$HPO$_4$ and 70mM NaH$_2$PO$_4$, sterile) are mixed and the DNA/CaCl$_2$ solution is slowly added dropwise while extensively bubbling in with a Pasteur pipette. This mixture is then left at room temperature for 30 min (Graham and Van der Eb, Virology, 52, 456; 1984).

### E.4.3.3 - Transfection of A1251 cells

A1251 cells are grown close to confluence in complete medium [DME medium (Gibco) supplemented with 10% fetal calf serum (Hyclone) and 1% antibiotic solution (Gibco)] in 5% CO$_2$ humified incubator at 37°C. 24 hours before transfection, the cells are splitted in 60mm Petri dishes at $10^5$ cell/dish and re-feed with fresh medium 4 to 6 hours before transfection. At t$_0$ the precipitated DNA is added and the mixture is left for 16 to 18 hours in the incubator. After overnight incubation, the cells are washed once with DME (7.2) then re-supplemented with the complete medium, left in the incubator to complete the incubation and harvested. In the experiments run in triplicate, all the steps following the dissolution of the dried DNA are performed separately for each sample. The harvested cells are used in the subsequent steps.

### E.4.3.4 - Preparation of cell lysates

64 ±4 hours after the addition of the precipitated DNA, the cells (prepared as in E 4.3.3.) are washed 3 times with a phosphate-buffered saline (PBS) free from Ca$^{++}$ (80 g NaCl, 2 g KCl, 11.5 g Na$_2$HPO$_4$.2H$_2$O, 2 g KH$_2$PO$_4$ per 1 liter of distilled water, brought to pH 7.2) then 1ml of STE (100mM NaCl, 10mM TRIS, 1mM EDTA) is added and the mixture is left at room temperature for 5 min. Afterwards the cells are scraped off with a rubber policeman and put in an Eppendorf tube, spinned down in microcentrifuge 3 min at 4°C and resuspended in 100 microl of Tris 250mM pH 7.8. The cell suspension is left 15 min in a dry ice/ethanol bath and then transferred to a 37°C bath; this freeze/thaw cycle is repeated twice, the cell nuclei are spinned down in microcentrifuge at 4°C for 5 min and the supernatant is transferred to a fresh tube. In order to overcome minor differences in the protein content of each sample due to a possible slight difference in cell number from plate to plate, protein concentration is determined by evaluating 10 microl from each sample according to the method of Lowry (see E.1.6). The remaining part of the lysates are stored at -20°C until used in the CAT assay.

### E4.3.5 - CAT assay

A volume containing 100 microg of protein from each lysate is brought to 133.5 microl with water and 44 microl of a premixed solution containing 22.5 microl of 2M Tris pH 7.8, 20 microl of a freshly prepared solution of 3 mg/ml acetylCoA (Sigma) and 1.5 microl radioactive $^{14}$C-chloramphenicol (50-60 mCi/mmol, from Amersham) are added thereto. The mixture is incubated at 37°C for 1 h, then the reaction is stopped by adding 1ml of ethyl acetate and vortexing. The two phases which form are separated in an Eppendorf microcentrifuge (5 min) and the organic phase is transferred to a fresh Eppendorf tube, essicated in a Savant vacuum centrifuge, redissolved in 20 microl of ethyl acetate and assayed by TLC on 0.25-1 mm silica gel plates (Kodak). The chromatography is run for 2-3 h with chloroform:methanol (HPLC grade), 95:5 as the mobile phase, and the radioactive spots corresponding to the labelled antibiotic are visualized by autoradiography. The areas of the chromatogram corresponding to the radioactive spots are cut out, placed in a vial with 5ml of a suitable scintillation solution such as Econofluor, (New England Nuclear) and the radioactivity is measured in a beta-counter. The results are shown in Tab. 3 below.

### E4.4.1 - Construction of plasmid puPA2/17.

300ng of puPA2/41 DNA (see E3.2) is completely digested with 4.2 U of OxaNI for 4 h at 37°C in the appropriate buffer (100 mM NaCl, 10mM Tris.HCl pH 7.5, 10 mM MgCl$_2$, 10 mM 2-mercaptoethanol, 100 microg/ml BSA). DNA restriction is checked on an ethidium bromide containing minigel (1.5.1) and the enzyme is inactivated by heating 10 min at 68°C. 2 microl of water, 1.5 microl of 10X buffer (see 1.5.5) and 1.5 microl of T4 DNA ligase are then added and the mixture incubated at 15°C for 18 h. 2.5 Microl of this mixture are used to transform E.Coli HB101 competent cells (1.5.8). Ampicillin resistant colonies are analyzed by miniprep analysis (1.5.9). Those having the correct restriction pattern are selected and one of them was named puPA2/17. (Fig. 8).

### E4.4.2 - Construction of plasmid puPA2/254.

4 microg of puPA2/41 DNA (see Fig.8) is digested to completion with 5U of EcoRV in 10 microl of the appropriate buffer (50 mMNaCl, 10mM Tris HCl, 10 mM MgCl$_2$ and 1mM dithiotreitol); (Maniatis et al) at 37°C for 2, h then a mixture of 7 microl of water, 2 microl of 0.5 M Tris pH8 and 1U CIP in 1 microl of buffer (Maniatis et al) is added. The mixture is incubated 1 h at 37°C, EGTA is added to a final concentration of 10mM and the mixture is incubated at 68°C for 45 min. The DNA is precipitated, washed and dried as described in 1.5.3 and finally redissolved in 7.5 microl TE (Maniatis et al). The DNA is completely digested with 4.2 U of OxaNI for 4 h at 37°C in 10 microl (final volume) and the 5′ ends are filled in with 1 U of the Klenow fragment of DNA Poll (20 microl, final volume) at 37°C for 1 h. The DNA is then precipitated (1.5.3) and resuspended in 7.5 microl TE (Maniatis et al.). 0.5 microl of 100mM ATP, l microl 10X buffer (see 1.5.5) (Maniatis et al) and 2.5U of T4 DNA ligase (1 microl, final volume) are added and the mixture is incubated for 18 h at 15°C. Then 2.5 microl of this mixture is used to transform E.Coli HB101 competent cells (1.5.8), the bacteria are plated on ampicillin containing LB plates (Maniatis et al). The colonies having the correct restriction pattern are selected and one of them was named puPA2/254 (Fig. 8).

### E4.4.3 - CAT analysis.

puPA2/41, its deletion derivatives puPA2/17 and puPA2/254 as well as RSV-CAT are tested for their ability to drive the trascription of the CAT gene. Plasmid DNAs are transfected in A1251 cell lines (1.1) by means of the calcium phosphate precipitation technique and the CAT intracellular level is measured after 64 ±4 hours. Purification of plasmid DNA, precipitation by calcium phosphate, cell transfection, preparation of cell lysates and CAT assay are performed substantially as described in E4.3. The results are shown below:

TABLE

|  | A1251 |
|---|---|
| RSV-CAT | 100 |
| puPA2/41 | 128 |
| puPA2/17 | 303.8 |
| puPA2/254 | 378.5 |

### E4.5 - Construction of uPA derived expression vector.

The bacterial CAT gene may be excised from plasmids puPA2/41, puPA2/17 and puPA2/254 in order to use them as expression vectors. Preferentially, modifications will conserve the uPA promoter derived sequence, the uPA TATA box, the mRNA starting point, and the pEMBL8-derived polylinker. DNA fragments comprising these regions may be excised from the vector and inserted upstream to different genes as "expression cassettes". From the above described plasmids three uPA-derived expression vectors were obtained by exciding the bacterial CAT gene: pPP41 from puPA/41, pPP17 from puPA2/17; pPP254 from puPA2/254.

### E4.5.1 - Construction of pPP41

25 microg of puPA2/41 DNA are partially digested with 4U of XbaI at 37°C for 45 min and the reaction stopped adding EDTA (to 10mM) and heat inactivating. After precipitation (1.5.3), the DNA is filled in with 2.5U of the Klenow fragment of DNA PolI (final volume 20 microl) at 37°C for 1 h, ligated to a 50 molar excess of SalI linker (1.5.5 and 1.5.6) for 16 h at 8°C with 2U ligase and, after heat-inactivation of the enzyme, completely digested at 37°C for 2 h with an excess of SalI. DNA fragments are run on a 0.75 agarose gel (1.5.7) and the gel portion containing a DNA fragment of 5-6kb range is cut out. This DNA is then extracted and purified as described in 1.5.7, resuspended in 10 microl TE and 2 microl of this solution are self-ligated at 16°C for 4 h with 2U ligase (final volume 10 microl). 2.5 microl of this DNA preparation are then used to transform DH5 E. coli competent cells (1.5.8 - 1.5.9). Among the ampicillin-resistant colonies plasmid pPP41, i.e. a plasmid having the region comprised between the SalI site and the XbaI site downstream the CAT gene deleted, is selected by miniprep analysis (Fig. 9B). .

### E4.5.2 - Construction of pPP/17 and pPP/254

Substantially following the above outlined procedure, two expression vectors are obtained by completely digesting 10 microg of puPA2/17 DNA or 10 microg of puPA2/254 DNA, respectively, with 24U XbaI at 37°C for 2 h. The 5' protruding ends of the linearized plasmid are filled in with the Klenow fragment of DNA PolI (2.5U) at 37°C for 1 h and then ligated to a 50 molar excess of SalI linker (1.5.5 and 1.5.6) for 16 h at 8°C with 2U ligase and, after heat-inactivation of the enzyme, the DNA is completely digested at 37°C for 2 h with an excess of SalI. Then, this enzyme is heat-inactivated and the DNA is precipitated (1.5.3) and re-suspended in 10 microl TE. 2 microl of this DNA solution are ligated at 15°C for 16 h with 2U DNA ligase (final volume 10 microl) and 2.5 microl are used to transform DH5 E.Coli competent cells. Among the ampicillin resistant colonies, plasmid pPP17 and pPP254 are selected which carry uPA-derived deletion promoters and have the same general characteristics described for pPP41 in E4.5.1 (Fig. 9B).

Example E5

Preparation of uPA derived minigenes.

E5.1. - Preparation of vectors.

Plasmid DNAs from pPP41, pPP17, pPP254 (20 microg each, prepared as described above) are linearized by separately digesting them with 50 U of SmaI at 25°C for 3 h (final volume 50 microl). Then the linearized plasmids are dephosphorilated with 1U of CIP at 37°C for 1 h, purified on 0.8% agarose gel (see 1.5.3), resuspended in 20 microl TE and stored at 4°C.

E5.2 - Preparation of the minigenes.

Each linearized dephosphorilated plasmid DNA obtained above (E5.1) (3 microg) is mixed with 1 microg of uPA-ORF (i.e. SmaI fragment (7.3 kb) containing the uPA coding region obtained as described in E1.1) in 10% PEG 1500 with 2U ligase (final volume 10 microl) for 16 h at 15°C.

2.5 microl from each reaction mixture is used to transform competent E. Coli DH5 cells (c.f. 1.5.8). Among the ampicillin-resistant colonies plasmid pPP41XS, pPP17XS, and pPP254XS, which derive from pPP41, pPP17 and pPP254, respectively, are selected by miniprep analysis (c.f. 1.5.9) for having the following characteristics (Fig. 9C):

- the uPA coding region is inserted in the SmaI site of the uPA promoter containing vector
- the uPA mRNA starting site is the same as the human uPA gene
- they contain a pEMBL8 derived sequence
- the uPA-derived minigenes can be excised from that vectors and inserted in different vectors preferably by digestion with XhoI and SalI
- the excised minigenes can be inserted in vectors having either a SalI or a XhoI site
- the excised minigenes can be inserted in vectors able to maintain them in an episomal form when inserted in a suitable host
- pPP41XS has the uPA coding region under the control of a 2.35 fragment from the uPA promoter;
- pPP17XS has the uPA coding region under the control of the same promoter fragment contained in pPP41XS but in which a deletion has been created between the two OxaNI sites;
- pPP254XS has the uPA coding region under the control of same promoter fragment contained in pPP41XS but in which a deletion has been created between the 5' OxaNI site and the EcoRV site.

E.5.4. - Trasfection/mass cultivation

Transfection experiments in murine LB6 cells are performed as described under E.1.4.1. The highest producing cells are mass cultivated as indicated in E.1.4.2.

E.5.5. - Quantitation of uPA

The amount of uPA produced by the cells transfected according to E.5.4. is evaluated as indicated under E.1.5. The results of transfections made co-transfecting the plasmid containing the gene for the resistance to neomycin (neo) under the control of the long terminal repeats of the Rous Sarcoma Virus (RSV-LTR) and the plasmid containing the uPA gene under the control of a uPA-derived promoter (pPP41XS, pPP17XS or pPP254XS) are reported below (secreted pro-uPA is evaluated by IAA, c.f. 1.6.2; pro-uPA levels from the RSV-uPA transformed clone is considered as 100%):

34

|  |  | % pro-uPA production |
|---|---|---|
| RSVuPA |  | 100 |
| pPP41XS:clone | 1 | 73 |
|  | 2 | 62 |
|  | 3 | 63 |
| pPP17XS:clone | 1 | 212 |
|  | 2 | 202 |
|  | 3 | 183 |
| pPP254XS:clone | 1 | 209 |
|  | 2 | 178 |
|  |  | 231 |

The physico-chemical and biological characteristics of the recovered scu-PA are indistinguishible from those of RSV-LB6-scu-PA (3F11 scuPA) reported above.

By repeating the transfection and mass cultivation in CHO or A431 cells, a scu-PA is obtained with the same chimico-physical and biological features of RSV-CHO-scu-PA or RSV-A431-scuPA, respectively

## Claims

1. A method for preparing human-type single chain glycosylated urokinase (pro-uPA) which comprises transforming an animal cell with an expression vector containing the uPA coding genomic sequence.

2. A method according to claim 1 wherein the uPA genomic sequence is a DNA fragment of about 7.3 kb obtained by partial SmaI digestion of a genomic DNA cloned into a suitable cloning vector.

3. A method according to claim 2 wherein the cloning vector is bacteriophage lambda.

4. A method according to claims 1, 2 or 3, wherein the animal cells are mammalian cells.

5. A method according to claim 1, 2, 3 or 4, wherein the animal cells are selected from established human, murine, hamster, and monkey cell lines.

6. A method according to claim 5 wherein the cell line is selected from murine LB6, Chinese hamster ovary (CHO) and human epidermoid carcinoma cell lines.

7. A method according to claim 1, 2, 3, 4, 5, or 6 wherein the uPA coding sequence is under the control of the human uPA promoter or a derivative thereof of human origin.

8. A method according to claim 7 wherein the human uPA promoter has the sequence represented in Fig. 7 or an allelic form thereof.

9. A method according to claim 7 wherein the human uPA promoter is a 2.38 Kb fragment obtained by SmaI digestion of a genomic DNA cloned into a suitable cloning vector.

10. A method according to claim 9 wherein the cloning vector is bacteriophage lambda.

11. A method according to any of claims 1 to 10 wherein the promoter is a deletion derivative of the human uPA promoter.

12. A method according to claim 11 wherein the human uPA promoter derivative is a deletion derivative missing the 0.6 Kb fragment cut by OxaNI between about - 1827 bp and -1202 bp or a deletion derivative missing the 1.29 Kb fragment between the OxaNI site at about - 1827 bp and the EcoRV site at about - 537 bp.

13. A promoter sequence for use in expressing a coding sequence in mammalian cells which is an insertion, deletion or mutation derivative of the human uPA promoter.

14. A promoter sequence of claim 13 which is a deletion derivative of a human uPA promoter.

15. A promoter sequence according to claim 13 wherein the human uPA promoter derivative is a deletion derivative missing the 0.6 Kb fragment cut by OxaNI or a deletion derivative missing the 1.29 Kb fragment between the OxaNI site at about - 1827 bp and the EcoRV site at about - 597 bp.

16. An expression cassette comprising a promoter of claims 13, 14 or 15.

17. An expression vector capable of transcribing a flanking gene or cDNA in eukaryotic cells which comprises a human uPA promoter sequence or a promoter of claims 13, 14 or 15.

18. An expression vector having a DNA sequence which comprises the uPA coding human genomic sequence and a promoter sequence which is a human uPA promoter sequence or a promoter according to claims 13, 14 or 15.

19. A plasmid which is selected from pPP41, pPP17, and pPP254.

20. A plasmid capable of expressing the human uPA coding sequence in mammalian cells which is selected from pPP41XS, pPP17XS, and pPP254XS.

21. A transformed animal cell which contains a promoter of claim 13, 14 or 15 or a plasmid of claims 19 or 20.

22. A transformed cell according to claim 21 which is capable of expressing uPA or scu-PA.

23. A transformed cell according to claims 21 or 22 which is selected from established human, murine, hamster and monkey cell lines.

24. A transformed cell according to claims 21 or 22 which is selected from murine LB6, Chinese hamster ovary and human epidermoid carcinoma cell lines.

25. A transformed cell according to claims 21 or 22 which is a Chinese hamster ovary cell line capable of growing and expressing the protein in suspension in the growth medium.

26. A transformed cell according to any one of claims 21, 22, 23, 24 or 25 which contains a resistance gene to a selection agent.

27. Glycosylated single-chain human-type urinary plasminogen activator having an apparent molecular weight in gel electrophoresis analysis of 500-3000 units higher than scu-PA obtained from human urine, said difference being due to a difference in molecular weight depending on the different glycosilation of the B-chain.

28. Glycosylated single-chain human urokinase-type plasminogen activator having:

a) the amino and carboxy terminal sequences of human pro-uPA

b) an apparent molecular weight of about 52,000 in SDS-PAGE, corresponding to a molecular weight of about 1000-2000 units higher than human urine obtained pro-uPA, determined under the same assay conditions; said difference in molecular weight being essentially due to a corresponding difference in the molecular weight of the B-chain

c) an apparent molecular weight of the A-chain equal to human A chain

d) an apparent molecular weight of the B-chain equal to that of B-chain of human origin after removal of N-glycosidic substituents by glycopeptidase F.

29. Glycosylated single-chain human urokinase-type plasminogen activator according to claims 27 or 28 further characterized in that it is produced by transformed murine LB6, Chinese hamster ovary or human epidermoid carcinoma cell lines.

30. Glycosylated single-chain human urokinase-type plasminogen activator according to claim 29 wherein the produced cell lines are transformed according to the process of claim 7.

31. Glycosylated single chain human urokinase-type plasminogen activator having the characteristics of 3F11-sc-uPA, RSV-A431-scu-PA, RSV-CHO-scu-PA or BPV-LB6-scu-PA

32. Human-type two-chain urokinase obtained by proteolytic cleavage of glycosylated, single chain, human-type pro-uPA of claims 27, 28, 29, 30 or 31 or prepared according to the method of any one of claims 1 to 12.

33. A pharmaceutical composition containing a compound of claim 27, 28, 29, 30 or 31 as the active ingredient.


Claims for the following Contracting States : ES, GR

1. A method for preparing human-type single chain glycosylated urokinase (pro-uPA) which comprises transforming an animal cell with an expression vector containing the uPA coding genomic sequence.

2. A method according to claim 1 wherein the uPA genomic sequence is a DNA fragment of about 7.3 kb obtained by partial SmaI digestion of a genomic DNA cloned into a suitable cloning vector.

3. A method according to claim 2 wherein the cloning vector is bacteriophage lambda.

4. A method according to claims 1, 2 or 3, wherein the animal cells are mammalian cells.

5. A method according to claim 1, 2, 3 or 4, wherein the animal cells are selected from established human, murine, hamster, and monkey cell lines.

6. A method according to claim 5 wherein the cell line is selected from murine LB6, Chinese hamster ovary (CHO) and human epidermoid carcinoma cell lines.

7. A method according to claim 1, 2, 3, 4, 5, or 6 wherein the uPA coding sequence is under the control of the human uPA promoter or a derivative thereof of human origin.

8. A method according to claim 7 wherein the human uPA promoter has the sequence represented in Fig. 7 or an allelic form thereof.

9. A method according to claim 7 wherein the human uPA promoter is a 2.38 Kb fragment obtained by SmaI digestion of a genomic DNA cloned into a suitable cloning vector.

10. A method according to claim 9 wherein the cloning vector is bacteriophage lambda.

11. A method according to any of claims 1 to 10 wherein the promoter is a deletion derivative of the human uPA promoter.

12. A method according to claim 11 wherein the human uPA promoter derivative is a deletion derivative missing the 0.6 Kb fragment cut by OxaNI between about - 1827 bp and -1202 bp or a deletion derivative missing the 1.29 Kb fragment between the OxaNI site at about - 1827 bp and the EcoRV site at about - 537 bp.

13. A method for preparing a promoter for use in expressing a coding sequence in mammalian cells which comprises modifying a human uPA promoter sequence by deletion, insertion or mutation.

14. A method according to claim 13 for preparing a promoter which is a deletion derivative of a human uPA promoter.

15. A method according to claim 13 for preparing a promoter which is a deletion derivative missing the 0.6 Kb fragment cut by OxaNI or a deletion derivative missing the 1.29 Kb fragment between the OxaNI site at about - 1827 bp and the EcoRV site at about - 597 bp.

16. A method for preparing an expression vector capable of transcribing a flanking gene or cDNA in eukariotic cells which comprises inserting a human uPA promoter sequence or a promoter of claims 13, 14 or 15 into a DNA vector in the proper orientation.

17. A method for preparing an expression vector capable of expression the uPA coding human genomic sequence in eukariotic cells which comprises inserting a promoter sequence which is a human uPA promoter sequence or a promoter according to claims 13, 14 or 15 upstream from a uPA coding human genomic sequence in the proper orientation.

18. A method according to claim 16 for preparing a plasmid having the features of pPP41, pPP17 or pPP254.

19. A method according to claim 17 for preparing a plasmid having the features of pPP41XS, pPP17XS or pPP254XS.

20. A method for preparing a transformed animal cell which comprises transforming a mammalian cell with a vector of claims 16, 17, 18 or 19.

21. A method according to claims 20 wherein the mammalian cell is selected from established human, murine, hamster and monkey cell lines.

22. A method according to claims 20 wherein the mammalian cell is selected from murine LB6, Chinese hamster ovary and human epidermoid carcinoma cell lines.

23. A method according to claims 20 wherein the mammalian cell is a Chinese hamster ovary cell line capable of growing and expressing the protein in suspension in the growth medium.

24. A method according to any one of claims 1 to 12 for preparing a glycosylated single-chain human-type urinary plasminogen activator having an apparent molecular weight of 500-3000 units higher than scu-PA obtained from human urine in gel electrophoresis analysis, said difference being due to a difference in molecular weight depeding on the different glycosilation of the of the B-chain.

25. A method according to any one claims 1 to 12 for preparing a glycosylated single-chain human urokinase-type plasminogen activator having:

    a) the amino and carboxy terminal sequences of human pro-uPA

    b) an apparent molecular weight of about 52,000 in SDS-PAGE, corresponding to a molecular weight of about 1000-2000 units higher than human urine obtained pro-uPA, determined under the same assay conditions; said difference in molecular weight being essentially due to a corresponding difference in the molecular weight of the B-chain

    c) an apparent molecular weight of the A-chain equal to human A chain

    d) an apparent molecular weight of the B-chain equal to that of B-chain of human origin after removal of N-glycosidic substituents by glycopeptidase F.

26. A method according to claims 24 or 25 further characterized in that the glycosylated single-chain human urokinase-type plasminogen activator is produced by a transformed murine LB6, Chinese hamster ovary or human epidermoid carcinoma cell line.

27. A method according to claims 24, 25 or 26 for preparing a glycosylated single chain human urokinase-type plasminogen activator having the characteristics of 3F11-sc-uPA, RSV-A431-scu-PA, RSV-CHO-scu-PA or BPV-LB6-scu-PA.

28. A method for preparing a human-type two-chain urokinase which comprises proteolytically cleaving a glycosylated, single chain, human-type pro-uPA of claims 24, 25, 26 or 27 or prepared according to the method of any one of claims 1 to 12.

phage λ-uPA2 insert

phage λ-uPA1 insert

uPA ORF

exon/intron structure

pHUK-1 cDNA

cDNA exon/protein structure

uPA protein/domains structure

Fig.1

Fig. 2

Fig. 3

pBPV 230.8

Sarver et al, Mol.Cell.Biol. 5, 3507 (1985)

Fig. 4

Fig. 5

Fig. 6

EP 0 303 028 A1

EP 0 303 028 A1

**Human uPA promoter sequence**

```
-2353                                                GGG GAGGCGGACG TTGCAGTGAG CCGAGATTGT GCCACTGCAC TCCAGCCTGG
-2300 GTGACAGACC GAGACTCCGT CTCAAAAAAA AAAAAAAAAA AATATGGCTG GGCGTGGTGG CTCATGCCTG TAATCCCAGC ACTTTGGAAG GATGAGGTGG
-2200 GAGGACCCCT TGAACCCAGA AGCCCAGCAA AACCTTGTCT TTAAAAAAAA AAAAGAACTG TGCACAAAGA TTTCAGAGAG TGCTAAAGAT TAGCGCATGG
-2100 ATAAGGAAGT TCTGTGAAGA GTTGAAGTGC TAGGTGAAGA GGTGGCACGG GGGAGGAGGG GGCGGAAGGG GAGAAAGGGT GTCACGCTTC ATAACGGTCT
-2000 CCAAACCTCT TTGTCCAGGA GGAAATGAAG TCATCTGTCC TCTCAGCAAT CAGCATGACA GCCTCCAGCC AAGTAACCCT GGAGTCATGA GAGCTGCTAG
-1900 GGGAGCAACA TGAATCATGA CGCGCCCCCT GGGAATTTCC TGATTACTAA CCTGGGAGTT TCGGGGTAAG TCCTCAGGCT GCAGCATCTC TGTTTATGTT
-1800 CTGGTCACGT TTATTTACAA TTAATGGGTT CTCAAATCCA AACAAAACTG ACCACAGTCT TCTAGAGGAA GTAGCAAGGT TGGCTCTGAA GCCTATAGCA
-1700 TCTCTGACTC AGTCTGTCCC CTGGAAGGCT GGCAGCTCAG CAAGCACAGA AGTCTCTCCA GAAGACAGTG GGTCACCTGC CTCCCAAAAG CTGAAAGACT
-1600 AACTTGTAAT TTCCCCAGCA GGCAGCTGGG ATCCTGAGCC CTCGGCTGGG GCAGAGCAAA GGAGCCTTCC TCCTTCCTAC CTTCCTGGCA CTCTCCCTGC
-1500 CTTCCTTCTG TCACTCTCAG GTGGACCCAG ACCCAAGGTC CAGATTTGCA AGGCAGGAAA ATGCTGCAGG CCTAGGCTGG GAAAGGGCCC AAAGCCGCTA
-1400 GTGGATTGCT GGGACTCAGC CTCCTCCTTC CCACTAAGAG AGCGAGTCGT ACTGGGTTCA AAATGACCCC ANSCCCTGGT TTCTGACACT AGGGGAAAGA
-1300 GATGGGCGTG ACAGAATCAC AGAAQCCCTG CTATGTTCGT CCAAGTGTGC CCAGAGATGC GTGTGTGTGT GTGTGTATAC ACAAATGTCT GCTTATCCTC
-1200 AGGCAGGAAG GGTGGATNCA GTCATTTACA CATGGTCTGT TTTTCTGGAG GACAATTTTA TTTGATAAAC AATTGTTTCT ATCTGAATAG AATAAACAAG
-1100 GCTCTATGAT GAAGTAAAAC ACTAAATACA CATGCATTAA AAAATGCATA ATTATCTTTT TGGAATGGGC TATACAGAGA TGTGCTTTTT AACCTGTTAA
-1000 GAGTGTAAAA GGACAAACAG TGAAAARTRA TRCRTCCTCT TAQTTTGTCC TCCAGTCTCC CANTTCCTCT ACTCAGNGGT GAGNGAGNCT TCCACACCCC
 -900 TCCAGAACCT CCACAGTTAG AACTGTCTAC ATGTTTCCCA TTGCTTTTAC TTTTATTCTT GCCTGCACAV ATAAATGAAT TGCTCCATTA TGGAAACTTC
 -800 CCAAAACGCA TCCGCGTTAA CACTTCAATA GGAAGCACCA ACAGTTTATG CCCTAGGCTT TGTTCCCACA ATCCTGTAAC ATCATATCAC GACACCTAAC
 -700 CCAATCCTTA TCAAGCCCTG TCAAAAACGG ACTTTAAACC AAGCTGCAAA TTTTCAGTAA TCTGGCCTTG CCTTTCCCCC TCTGATAGCA CCATCAAACA
 -600 AACCCCCTTA CTGCCGAAAG CAATAAGCCC GCCTTTGTTC CATCCACTGG TTGTGTTGGT GATATCTGGG GACTGCCACT GAACAGACGC ACAGAGGGAG
 -500 CCCCTACAGG CAGGGGTTTT TCTGTCTGTG CTTCTTGGGA GAGTATGTCT CGTACATTTG TCGCGTGATG AAGACTTCAC AGCTCCATCA GCTGCGGGCA
 -400 AGGGGGTCTG AGGCAGTCTT AGGCAAGTTG GGGCCCAGCG GGAGAAGTTG CAGAAGAACT GATTAGAGGA CCCCAGGAGG CTTCAGAGCT GGGCGAGGTA
 -300 GAGAGTCTCC TGTGCGCCTT CTCTCCTCTC TGCAATTCGG GGACTCCTTG CACTGGGGCA GGCCCCCGGC AGGTGCATGG GAGGAAGCAC GGAGAATTTA
 -200 CAAGCCTCTC GATTCCTCAG TCCAGACGCT GTTGGGTCCC CTCCGCTGGA GATCGCGCTT CCCCCAAATC TTTGTGAGCG TTGCGGAAGC ACGCGGGGTC
 -100 CGGGTCGCTG AGCGCTGCAA GACAGGGGAG GGAGCCGGGC GGGAGAGGGA GGGGCGGCGC CGGGGCGGGC CCTGATATAG AGCAGCCGCC GCGGGTCGCA
  +1 GCACAGTCGG AGACCGCAGC CCGGAGCCC
```

Fig. 7

Fig. 8

EP 0 303 028 A1

Fig. 9

AMINO ACID SEQUENCE

(Fig. 10)

|  | 1 | 5 | 10 | 15 |
|---|---|---|---|---|

H. kideny scu-PA  Ser-Asn-Glu-Leu-His-Gln-Val-Pro-Ser-Asn-Cys-Asp-Cys-Leu-Asn-Gly-Gly-Thr

H. A431 scu-PA  Ser-Asn-Glu-Leu-His-Gln-Val-Pro-Ser-Asn-Cys-Asp-Gls-Leu-Asn-Gly-Gly-Thr

H. urine scu-PA  Ser-Asn-Glu-Leu-His-Gln-Val-Pro-Ser-Asn-Cys-Asp-Cys-Leu-Asn-Gly-Gly-Thr

3F11 scu-PA  Ser-Asn-Glu-Leu-His-Gln-Val-Pro-Ser-Asn-Cys-Asp-Cys-Leu-Asn-Gly-Gly-Thr

Mouse scu-PA  Gly-Ser-Val-Leu-Gly-Ala-Pro-Asp-Glu-Ser-Asn-Cys-Gly-Cys-Gln-Asn-Gly-Gly

EP 0 303 028 A1

Fig. 11

Fig. 12

Fig. 13

Fig. 14

## DOCUMENTS CONSIDERED TO BE RELEVANT

EP 88109800.8

| Category | Citation of document with indication, where appropriate, of relevant passages | Relevant to claim | CLASSIFICATION OF THE APPLICATION (Int. Cl.4) |
|---|---|---|---|
| D,X | EP - A2 - 0 154 272 (THE GREEN CROSS CORPORATION)<br><br>* Claims; page 1 * | 1,4,5, 21-23, 27,28, 33 | C 12 N 15/00<br>C 12 N 9/72<br>C 07 H 21/04<br>C 12 N 5/00<br>A 61 K 37/54 |
| X | JP - A - 62-149 625 (publ. 03.07.87<br>& EP-A2-0 232 544 (GREEN CROSS CORPORATION)<br><br>* Abstract * | 1,4,5, 21-23 | |
| D,A | NUCLEIC ACIDS RESEARCH, vol. 13, no. 8, April 25, 1985 (Oxford, GB)<br>A.RICCIO et al. "The human uro-kinase-plasminogen activator gene and its promoter"<br>pages 2759-2771<br><br>* Totality * | 1,4,7 | |

TECHNICAL FIELDS SEARCHED (Int. Cl.4)

C 12 N

C 07 H

A 61 K

The present search report has been drawn up for all claims

| Place of search | Date of completion of the search | Examiner |
|---|---|---|
| VIENNA | 11-11-1988 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82